# EUROPEAN PATENT APPLICATION

(11) **EP 4 714 345 A1**
(43) Date of publication of application: **25.03.2026**
(21) Application number: 25197229.5
(22) Date of filing: 21.08.2025
(51) Int. Cl.: A61B 5/11, A61B 5/16, A61B 5/00, G16H 50/30

(54) **MEASUREMENT OF READINESS FOR DAILY ACTIVITIES USING WEARABLE DATA AND SUBJECTIVE INPUT**

(30) Priority: 30.08.2024 US 202418820790
(71) Applicant: Anhui Huami Health Technology Co., Ltd., Hefei, Anhui 230088 (CN)
(72) Inventor: HERZALLAH, Mohammad M., Cupertino, California 95014 (US); SHI, Mingshu, Hefei, 230088 (CN); TANG, Mengfan, Cupertino, California 95014 (US); WANG, Kongqiao, Cupertino, California 95014 (US)
(74) Representative: Dai, Simin

(57) **Abstract**

A method of dynamically determining a readiness score of a user using a wearable device. The method includes detecting, by the wearable device when worn by the user, one or more sensor or physiological signals associated with the user, and determining, by a processor, an estimation of two or more readiness components based on the one or more sensor or physiological signals. The two or more readiness components include a physical energy of the user associated with physical energy consumption of the user, physical energy recovery of the user, or both. The two or more readiness components also include a mental energy of the user associated with mental energy consumption of the user, mental energy recovery of the user, or both. The method further includes determining the readiness score based on the estimation of each of the two or more readiness components.

## Description

### TECHNICAL FIELD

This application relates to wearable computing, and in particular, to dynamic monitoring and measurement of a user's readiness for daily activities.

### BACKGROUND

Modern technologies have provided users with wearable computing devices configured to sense and track a user's physical parameters. Based upon such physical parameters of the user, the wearable computing devices of associated computer-based system may compute health-related analyses to track the health of the user.

Current wearable computing devices may also compute analyses to correlate the detected physical parameters of the user with health metrics of the user.

### SUMMARY

Disclosed herein are implementations of methods, apparatuses, and systems for measurement of readiness and subsequent guidance.

In one aspect of the disclosure, a method of dynamically determining a readiness score of a user using a wearable device is disclosed. The method includes: acquiring, by a processor, one or more sensor or physiological signals associated with the user, detected by the wearable device when worn by the user, and determining, by the processor, an estimation of two or more readiness components based on the one or more sensor or physiological signals. The two or more readiness components include a physical energy of the user associated with physical energy consumption of the user, physical energy recovery of the user, or both. The two or more readiness components also include a mental energy of the user associated with mental energy consumption of the user, mental energy recovery of the user, or both. The method further includes determining the readiness score based on the estimation of each of the two or more readiness components.

In some embodiments, the physical energy of the user may be determined based on a physical energy reserve of the user (such as physical energy of the user at a current or previous time point), one or more heart-related metrics of the user, and one or more motion-related metrics of the user.

In some examples, the one or more heart-related metrics may include a real-time heart rate reserve, a real-time heart rate, or both.

In some examples, the one or more motion-related metrics may include an activity level.

In some examples, at least one of the one or more heart-related metrics or the one or more motion-related metrics is obtained based on the one or more sensor or physiological signals.

In some embodiments, the one or more sensor or physiological signals include a pulse signal and a motion signal.

In some embodiments, at least one of a real-time heart rate or a real-time heart rate reserve of the user is obtained based on the pulse signal.

In some embodiments, an activity level of the user is obtained based on the motion signal, or based on the motion signal and the pulse signal.

In some embodiments, determining the estimation of the two or more readiness components based on the one or more sensor or physiological signals may include: determining at least one of a real-time heart rate, a real-time heart rate reserve or an activity level of the user based on the one or more sensor or physiological signals; and determining that the user is in a state of physical energy consumption or a state of physical energy recovery based on at least one of the real-time heart rate, the real-time heart rate reserve or the activity level of the user.

In some embodiments, determining the estimation of the two or more readiness components based on the one or more sensor or physiological signals may include: determining at least one of a real-time heart rate, a real-time heart rate reserve or an activity level of the user based on the one or more sensor or physiological signals; and determining or updating the physical energy of the user based on at least one of the real-time heart rate, the real-time heart time reserve or the activity level of the user.

In some embodiments, determining the estimation of the two or more readiness components based on the one or more sensor or physiological signals, includes: determining a real-time heart rate reserve of the user based on the one or more sensor or physiological signals; and determining or updating the physical energy of the user based on the real-time heart rate reserve of the user.

In some embodiments, determining the estimation of the two or more readiness components based on the one or more sensor or physiological signals, includes: determining a real-time heart rate reserve of the user based on the one or more sensor or physiological signals; and determining that the user is in a state of physical energy consumption or a state of physical energy recovery based on the real-time heart rate reserve of the user.

In some embodiments, the real-time heart rate reserve of the user is determined based on a real-time heart rate of the user as well as at least one of a maximum heart rate or a resting heart rate of the user. For example, the real-time heart rate reserve of the user may be based on a difference between the maximum heart rate and the resting heart rate of the user, a difference between the real-time heart rate and at least one of the maximum heart rate or the resting heart rate of the user, or both.

In some embodiments, determining the estimation of the two or more readiness components based on the one or more sensor or physiological signals, includes: determining, based on the one or more sensor or physiological signals, a physical energy recovery rate of the user at a current time point.

In some embodiments, in a case that the user is in the state of physical energy recovery, a physical energy recovery rate of the user is determined based on the one or more sensor or physiological signals. For instance, the physical energy recovery rate of the user is determined based on one or more heart-related metrics (e.g., real-time HRR), and/or one or more motion-related metrics (e.g., activity level) of the user.

In some embodiments, determining the estimation of the two or more readiness components based on the one or more sensor or physiological signals, includes: selecting, based on the one or more sensor or physiological signals, a corresponding physical energy recovery model from a plurality of preset physical energy recovery models, where the plurality of preset physical energy recovery models correspond to different physical energy recovery rates.

In some embodiments, in a case that the user is in the state of physical energy recovery, a corresponding physical energy recovery model is selected from a plurality of preset physical energy recovery models. For example, the corresponding physical energy recovery model is selected based on one or more heart-related metrics (e.g., real-time HRR), and/or one or more motion-related metrics (e.g., activity level) of the user.

In some embodiments, one or more heart-related metrics of the user at a current time point are determined based on the one or more sensor or physiological signals, and by comparing the one or more heart-related metrics of the user at the current time point with a corresponding threshold, the physical energy of the user is determined or updated.

In some embodiments, one or more heart-related metrics of the user at a current time point are determined based on the one or more sensor or physiological signals, and by comparing the one or more heart-related metrics of the user at the current time point with a corresponding threshold, whether the user is in a state of physical energy consumption or a state of physical energy recovery at the current time point is determined.

In some embodiments, one or more heart-related metrics of the user at a current time point are determined based on the one or more sensor or physiological signals, and by comparing the one or more heart-related metrics of the user at the current time point with a corresponding threshold, a physical energy recovery rate of the user at the current time point is determined.

In some embodiments, one or more heart-related metrics of the user at a current time point are determined based on the one or more sensor or physiological signals, and by comparing the one or more heart-related metrics of the user at the current time point with a corresponding threshold, a corresponding physical energy recovery model is selected from a plurality of preset physical energy recovery models.

In some embodiments, a second physical energy recovery rate associated with a case where the one or more heart-related metrics of the user are greater than or equal to a corresponding threshold is less than a first physical energy recovery rate associated with a case where the one or more heart-related metrics of the user are less than the corresponding threshold.

In some embodiments, based on the one or more sensor or physiological signals, one or more heart-related metrics and one or more motion-related metrics of the user at a current time point are determined, and based on the one or more heart-related metrics and the one or more motion-related metrics of the user at the current time point, the physical energy of the user are determined or updated, and/or, whether the user is in a state of physical energy consumption or a state of physical energy recovery at the current time point is determined.

In some examples, in response to determining that the real-time heart rate reserve of the user is less than a corresponding threshold, it is determined that the user is in a state of physical energy recovery at the current time point, or the physical energy of the user is determined or updated based on a first physical energy recovery model, or the physical energy of the user is determined or updated based on a first physical energy recovery rate.

In some examples, in a case that the real-time heart rate reserve of the user is greater than or equal to the corresponding threshold, the activity level of the user at the current time point is determined based on the one or more sensor or physiological signals. In this case, optionally, the physical energy of the user may be determined or updated based on the activity level of the user at the current time point. Optionally, it may be determined whether the user is in a state of physical energy consumption or a state of physical energy recovery based on the activity level of the user at the current time point.

In some examples, in response to determining that the real-time heart rate reserve of the user at the current time point is greater than or equal to the corresponding threshold, and the activity level of the user at the current time point is greater than or equal to the corresponding threshold, it is determined that the user is in a state of physical energy consumption, or the physical energy of the user is determined or updated based on a physical energy consumption model.

In some examples, in response to determining that the real-time heart rate reserve of the user at the current time point is less than the corresponding threshold, and/or the activity level of the user at the current time point is less than the corresponding threshold, it is determined that the user is in a state of physical energy recovery, or the physical energy of the user is determined or updated based on a physical energy recovery model.

In some examples, in response to determining that the real-time heart rate reserve of the user at the current time point is less than the corresponding threshold, the physical energy of the user is determined or updated based on a first physical energy recovery model or a first physical energy recovery rate.

In some examples, in response to determining that the real-time heart rate reserve of the user at the current time point is greater than or equal to the corresponding threshold, and the activity level of the user at the current time point is less than the corresponding threshold, the physical energy of the user is determined or updated based on a second physical energy recovery model or a second physical energy recovery rate.

In some examples, the second physical energy recovery rate or a physical energy recovery rate corresponding to the second physical energy recovery model is less than the first physical energy recovery rate or a physical energy recovery rate corresponding to the first physical energy recovery model.

In some examples, a physical energy recovery rate associated with a case where the real-time heart rate reserve is greater than or equal to the corresponding threshold is lower than a physical energy recovery rate associated with a case where the real-time heart rate reserve is less than the corresponding threshold.

In some embodiments, the corresponding physical energy recovery or consumption model or rate of the user is based on a difference between the one or more heart-related metrics (e.g., the real-time heart rate reserve) of the user and the corresponding threshold.

In some embodiments, the physical energy recovery or consumption rate of the user is based on the physical energy reserve of the user (e.g., the physical energy of the user at a previous time point).

In some embodiments, the mental energy of the user may be determined based at least on a real-time stress of the user obtained from the one or more sensor or physiological signals and a mental energy reserve of the user.

In some embodiments, determining the estimation of the two or more readiness components based on the one or more sensor or physiological signals may include: determining whether the user is in a state of sleeping or not based on the one or more sensor or physiological signals; and determining, based on whether the user is in the state of sleeping or not, that the user is in a state of mental energy consumption or a state of mental energy recovery.

In some embodiments, determining the estimation of the two or more readiness components based on the one or more sensor or physiological signals may include: determining whether the user is in a state of sleeping or not based on the one or more sensor or physiological signals; and determining or updating the mental energy of the user based on whether the user is in the state of sleeping or not.

In some embodiments, determining the estimation of the two or more readiness components based on the one or more sensor or physiological signals may include: determining whether the user is in a state of mental energy consumption or a state of mental energy recovery based at least in part on a stress level associated with a severity of stress obtained from the one or more sensor or physiological signals.

In some embodiment, determining the estimation of the two or more readiness components based on the one or more sensor or physiological signals includes: determining a real-time stress value of the user at a current time point based on the one or more sensor or physiological signals; and adjusting the real-time stress value of the user at the current time point by comparing the real-time stress value of the user at the current time point and a real-time stress value of the user at at least one previous time point.

In some embodiment, determining the estimation of the two or more readiness components based on the one or more sensor or physiological signals includes: determining, based on the one or more sensor or physiological signals, whether the user is in a state of sleeping or not and a real-time stress value of the user; and determining, based on whether the user is in the state of sleeping or not and the real-time stress value of the user, that the user is in a state of mental energy consumption or a state of mental energy recovery.

In some embodiment, determining the estimation of the two or more readiness components based on the one or more sensor or physiological signals includes: determining, based on the one or more sensor or physiological signals, whether the user is in a state of sleeping or not and a real-time stress value of the user; and determining or updating the mental energy of the user based on whether the user is in the state of sleeping or not and the real-time stress value of the user.

In some embodiments, determining the estimation of the two or more readiness components based on the one or more sensor or physiological signals includes: determining a mental energy recovery rate of the user at a current time point based on the one or more sensor or physiological signals.

In some embodiments, determining the estimation of the two or more readiness components based on the one or more sensor or physiological signals includes: determining a corresponding mental energy recovery model from a plurality of preset mental energy recovery models based on the one or more sensor or physiological signals, where the plurality of preset mental energy recovery models correspond to different mental energy recovery rates.

In some embodiments, based on the one or more sensor or physiological signals, a cognitive reserve of the user at a current time point may be determined or updated, and based on the cognitive reserve of the user at the current time point, the mental energy of the user at the current time point may be determined.

In some embodiments, based on the one or more sensor or physiological signals, a sleep intensity of the user may be determined, and based on the sleep intensity of the user, the cognitive reserve and/or the mental energy of the user may be determined or updated.

In some embodiments, based on the one or more sensor or physiological signals, a sleep intensity and a real-time stress value of the user may be determined, and based on the sleep intensity and the real-time stress value of the user, the cognitive reserve and/or the mental energy of the user may be determined or updated.

In some embodiments, based on whether the user is in the state of sleeping or not, a mental energy recovery rate of the user is determined.

In some embodiments, based on whether the user is in the state of sleeping or not, a corresponding mental energy recovery model is determined from a plurality of preset mental energy recovery models.

In some embodiments, based on an estimation of the cognitive reserve of the user at the current time point, it is determined whether the user is in the state of sleeping or not.

In some embodiments, based on the cognitive reserve and the sleep intensity of the user, it is determined whether the user is in the state of sleeping or not.

In some embodiments, in response to determining that the user is in the state of sleeping, it is determined that the user is in a state of mental energy recovery.

In some embodiments, in response to determining that the user is in the state of sleeping, the mental energy of the user is determined or updated based on a first mental energy recovery model.

In some embodiments, in response to determining that the user is not in the state of sleeping, a real-time stress value of the user at the current time point is determined based on the one or more sensor or physiological signals. In this case, optionally, the mental energy of the user may be determined or updated based on the real-time stress value of the user at the current time point. Optionally, it may be determined whether the user is in a state of mental energy recovery or a state of mental energy consumption based on the real-time stress value of the user at the current time point.

In some embodiments, it may be determined whether the user is in a state of mental energy recovery or a state of mental energy consumption by comparing the real-time stress value of the user at the current time point with a corresponding stress threshold.

In some embodiments, in response to determining that the user is in the state of sleeping, and/or determining that the real-time stress value of the user at the current time point is less than the corresponding stress threshold, it is determined that the user is in the state of mental energy recovery.

In some embodiments, in response to determining that the user is not in the state of sleeping, and that the real-time stress value of the user at the current time point is greater than or equal to the corresponding stress threshold, it is determined that the user is in the state of mental energy consumption.

In some embodiments, in response to determining that the user is not in the state of sleeping, and that the real-time stress value of the user at the current time point is greater than or equal to the corresponding stress threshold, the mental energy of the user is determined or updated based on a second mental energy recovery model.

In some embodiments, a second mental energy recovery rate corresponding to the second mental energy recovery model is less than a first mental energy recovery rate corresponding to a first mental energy recovery model.

In some embodiments, a mental energy recovery rate associated with a case where the user is in the state of sleeping is greater than a mental energy recovery rate associated with a case where the user is not in the state of sleeping.

In some embodiments, the corresponding mental energy recovery or consumption model or rate of the user is based on a difference between the real-time stress value of the user at the current time point and the corresponding stress threshold.

In some embodiments, an assessment of heart health of the user (e.g., a heart health component score) is based on at least one of resting heart rate or heart rate variability of the user.

For example, based on the one or more sensor or physiological signals, at least one of the resting heart rate or the heart rate variability of the user may be determined, and based on at least one of the resting heart rate or the heart rate variability of the user, the component score for heart health of the user may be determined, or the assessment of the heart health of the user may be obtained.

In some embodiments, an assessment of breathing health of the user (e.g., a breathing health component score) is based on at least one of fluctuations in oxygen saturation or apnea-hypopnea index (AHI) of the user. For example, based on the one or more sensor or physiological signals, at least one of the oxygen saturation or the AHI of the user may be determined, and based on at least one of the oxygen saturation or the AHI of the user, a component score for breathing health of the user may be determined, or the assessment of the breathing health of the user may be obtained.

In some embodiments, based on at least one of user historical data or domain knowledge boundaries associated with a readiness component, a readiness component score of the readiness component is determined.

In some embodiments, user historical data associated with a readiness component is acquired and evaluated. Based on the evaluation of the user historical data, it is determined whether to use the user historical data or the domain knowledge boundaries associated with the readiness component to determine the readiness component score of the readiness component.

Optionally, by determining at least one of whether the number of historical data points included in the user historical data associated with a readiness component is sufficient or whether a statistic metric of the user historical data falls within a normal range, the user historical data or the domain knowledge boundaries is selected to determine the readiness component score of the readiness component.

In some embodiments, based on a statistic metric of the user historical data associated with the readiness components or a statistic metric of the domain knowledge boundaries associated with the readiness components, the readiness component scores corresponding to the readiness components are determined.

In some embodiments, the method may further include providing the readiness score and information associated with the estimation of each of the two or more readiness components to the user.

In some embodiments, the two or more readiness components may further include at least one of heart health, breathing health, and body temperature.

In some embodiments, the estimation of each of the two or more readiness components may be determined based on historical sensor or physiological data such that the estimation of each of the two or more readiness components is individualized for the user. The historical sensor or physiological data may be tracked based one or more timescales, the one or more timescales including at least one of a daily timescale, a weekly timescale, and a monthly timescale.

In some embodiments, the readiness score may be determined based on the estimations of the two or more readiness components as well as weights of the two or more readiness components. The weights of the two of more readiness components may be determined based on the estimations for the two or more readiness components.

In some embodiments, the method may further include: acquiring, by the processor, a subjective estimation of a readiness score and/or one or more readiness components input by the user.

In some embodiments, the method may further include: adjusting, by the processor, the readiness score based on the subjective estimation of the readiness score.

In some embodiments, the method may further include: adjusting, by the processor, the readiness score and/or one or more readiness component scores based on the subjective estimation of the one or more readiness components.

In some embodiments, the readiness score of the user may be determined for a current day based on the estimation of each of the two or more readiness components determined on a previous day. The readiness score may be updated in real-time based on continuous monitoring of the one or more sensor or physiological signals associated with the user.

In some embodiments, the method may further include providing guidance to the user based on the readiness score. The guidance may include at least one of information associated with the readiness score, information associated with the estimation of each of the two or more readiness components, instructions on how to address an underlying situation associated with the readiness score, or recommended activities.

In another aspect of the disclosure, an electronic device for dynamically determining a readiness score of a user using a wearable device is disclosed. The electronic device includes a non-transitory memory and a processor configured to execute instructions stored in the non-transitory memory to implement the method of any one of the above embodiments.

In some embodiments, the electronic device is the wearable device, and the electronic device further includes: at least one sensor configured to detect the one or more sensor or physiological signals associated with the user when the wearable device is worn by the user.

In another aspect of the disclosure, a non-transitory computer-readable storage medium configured to store computer programs for dynamically determining a readiness score of a user using a wearable device is disclosed. The computer programs include instructions executable by a processor to implement the method of any one of the above embodiments.

### BRIEF DESCRIPTION OF DRAWINGS

FIG. 1 is a perspective view of an example of a wearable device in accordance with the present teachings.
FIG. 2 is a block diagram of an example of a computing device that may be used with or incorporated into a wearable device in accordance with the present teachings.
FIG. 3 is a block diagram illustrating components utilized to determine a readiness score in accordance with the present teachings.
FIG. 4 is a flow diagram of an example of a method for dynamically determining physical energy of a user.
FIG. 5 is a flow diagram of an example of a method for dynamically determining mental energy of a user.
FIG. 6 is a flow diagram of an example of a method for dynamically determining a readiness component score in accordance with the present teachings.
FIG. 7 is a flow diagram of an example of a method for dynamically determining a readiness score of a user using a wearable device.

### DETAILED DESCRIPTION

Many portable devices and systems have been developed to monitor physiological conditions of an individual. One area of interest in the use of physiological monitors is personal wellness and mental health. Personal wellness and mental health are integral parts of the overall health of an individual and may be evaluated to determine an overall state of the individual at a given point in time. For example, personal wellness and mental health may be used to determine if an individual is physically and/or mentally ready for a particular task or activity at a given point in time. That is, the personal and mental wellness of an individual may be used (e.g., evaluated) to assess an overall readiness of an individual to partake and/or complete a particular task or activity. By way of example, the personal and mental wellness of an individual may be evaluated to determine the readiness of the individual to complete an exercise, a task at work, other activities, or any combination thereof.

The readiness of an individual to partake and/or complete a particular activity at a given point in time may be determined by obtaining one or more physical parameters of an individual (also referred to as a user herein) and associating such physical parameters with a readiness of the individual. By way of example, one or more physiological conditions of an individual (e.g., heart rate, heart rate variability (HRV), skin conductance, respiration rate, blood pressure, pupil dilation, body temperature, other physiological parameters, etc.) may be obtained by a device worn by the user and the physiological conditions may be evaluated to generically determine a readiness (e.g., a readiness value or readiness score) of the individual. The readiness of the individual may be associated with an overall energy level of the individual at a given point in time. However, such portable devices and systems thereof have certain shortcomings that hinder their overall usability. Similarly, such a generic determination of a readiness of an individual has certain shortcomings that hinder the readiness score from being particular useful and/or insightful for the individual.

The present teachings provide an evaluation system, which may be used by or in conjunction with one or more portable devices (e.g., wearable devices), that dynamically monitors and tracks a readiness of an individual to partake in any given activity and/or task at a particular point in time. The present teachings may dynamically determine energy consumption and/or recovery of an individual based upon daily activities. For example, the present teachings may dynamically determine an individual's overall energy change from a previous day (e.g., based on activities completed in the previous day) to provide the individual with a readiness score, whereby the individual may utilize the readiness score to modify their daily activities and account for necessary recovery and avoid excessive fatigue (i.e., burn-out). Moreover, the present teachings determine a readiness score for an individual based upon various components (e.g., physical recovery, mental recovery, heart health, breathing health, body temperature, etc.) that may contribute to the readiness of the individual, whereby the various components may be determined (e.g., assessed) based upon the one or more physiological conditions obtained by the wearable device. Furthermore, the readiness score may also provide an individualized assessment to the individual that combines both the aforementioned physiological components that contribute to the readiness of the individual and the individual's subjective input.

Based on the above, the present disclosure provides a data-drive approach that utilizes data from a wearable device (e.g., a smart watch worn by the individual) to determine and assess a readiness of an individual that could positively or negatively affect an individual's overall ability to complete particular activities and/or tasks in a given day. Implementations of this disclosure aim to help the individual successfully maintain a healthy physical and mental state and avoid burn-out caused by over-exertion. For example, based on the physiological conditions measured by the wearable device and the subjective feedback provided by the individual, the readiness of the individual may be determined and provided to the individual to help the individual optimize their daily activities to thereby prevent over-exertion of the individual both physically and mentally. That is, the present disclosure provides an evaluation system that may be an evaluation and guidance system for the physical and mental health of the individual.

In some embodiments, by dynamically monitoring physiological conditions of the individual and optionally receiving feedback directly from the individual (e.g., subjective feedback from the individual, which may be used to cross-validate and/or adjust the evaluation of the physiological conditions detected by the wearable device), the overall accuracy of the determined readiness score may be improved and more tailored to the individual. The readiness score may also be dynamically adjusted on a frequent and/or regular basis as needed to account for any discrepancies between the readiness determined by the evaluation system described herein and the readiness subjectively sensed by the individual. Thus, the readiness score provided to the individual may be tailored and personalized specifically to the individual and may produce a holistic view and assessment for the physical and/or mental health (e.g., physical and/or mental energy levels) of the individual that account for both objective data (e.g., the readiness determined based upon the physiological conditions detected by the wearable device) and subjective data (e.g., feedback from the individual).

Moreover, according to some implementations of this disclosure, sensor data for the individual can be collected, for example, by the portable device (e.g., the wearable device). The collected sensor data for the individual may be extracted to obtain physiological conditions of the individual, such as heart rate, heart rate variability (HRV), sleep analyses (such as sleep or not, sleep duration, sleep interruptions, sleep quality, or the like), exercise analyses (such as exercise intensity, exercise duration, exercise type, energy consumption during exercise, or the like), body temperature, skin conductance, respiration rate, blood pressure, pupil dilation, physiological stress level (e.g., a severity of physiological stress), an electrocardiogram (ECG), an electroencephalogram (EEG), an electrooculogram (EOG), an electromyogram (EMG), an electrodermal activity (EDA), other physiological parameters, or any combination thereof. Based upon the data extracted and physiological conditions determined, the readiness of the individual (e.g., the readiness of the individual to partake in and/or complete a given activity and/or task, whereby the readiness may be based on the physical and/or mental energy levels of the individual) may be accurately determined, which may be utilized by the individual to plan their daily activities and maintain a healthier overall physical and mental wellbeing, or may be utilized to plan or adjust training schedules for the individual, or may be utilized to provide the individual with recommendations for adjusting or improving their readiness, such as recommendations on whether or not to engage in a particular activity.

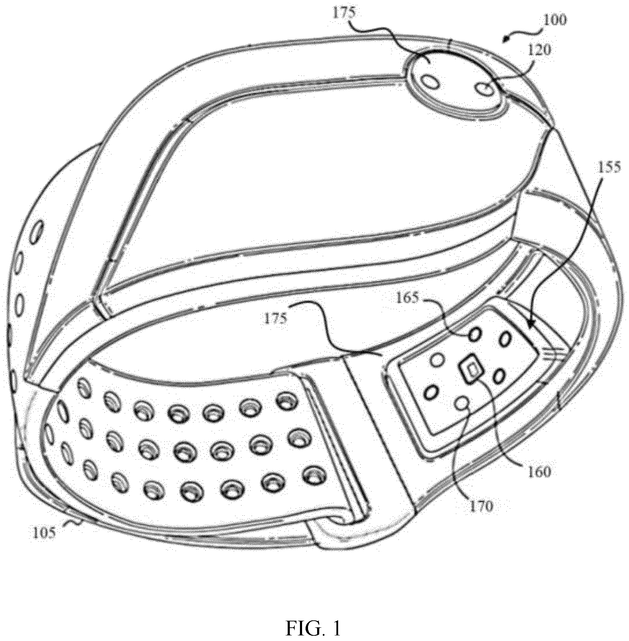

FIG. 1 depicts a perspective view of an example device 100 according to some implementations of this disclosure. The device 100 may be a wearable device worn by an individual (also referred to herein as a user) to at least one of sense, collect, monitor, analyze, or display information pertaining to one or more of a physiological parameter and/or motion parameter of the individual or an environmental parameter captured by the device 100 in a vicinity of the individual. The device 100 can include, for example, a head mounted device, a wrist mounted device, a ring, a strap (e.g., a chest strap, arm strap, leg strap, etc.), or headphones. Although depicted in FIG. 1 as a smart wristwatch, the device 100 can include the wearable device configured for positioning at a user's wrist, arm, finger, chest, leg, head, feet, torso, another extremity of the user, or some other area of the user's body, or the wearable device configured for positioning at a user's wearable clothing. In some embodiments, the device 100 can also be implemented as a wearable camera having an image sensor with capabilities such as high-speed shooting performance, low-light or dark shooting performance, high image quality, or anti-shake performance, among other things. In addition, the wearable camera can be equipped with other sensors (e.g., a photoplethysmography (PPG) sensor to detect heart rate, a blood pressure sensor, an ECG sensor, a positioning sensor, an altitude sensor, a temperature sensor, a humidity sensor, etc.).

In some embodiments, the device 100 may include one or more sensors and processing tools for detecting, collecting, processing, or displaying one or more physiological parameters of the individual and/or other information that may or may not be related to health (e.g., mental health and physical health), exercise, sleep, or physical training sessions (e.g., characteristic information, education information, etc.). In some examples, the physiological parameters can include at least one of: heart rate, heart rate variability (HRV), blood pressure, blood glucose level, oxygen saturation, respiration rate, body temperature, electrocardiogram (ECG) measurements, electroencephalogram (EEG) measurements, electrooculogram (EOG) readings, electromyogram (EMG) readings, electrodermal activity (EDA) readings, sleep parameters (e.g., one or any combination of sleep state, sleep phase, sleep quality, sleep time duration, sleep regularity, sleep staging data, a proportion of deep sleep, sleep interruptions, wakefulness after sleep, etc.), mental state, emotion, physiological stress state, and other physiological information that can be measured for the individual.

In some examples, the device 100 may include a motion sensor and a physiological sensor, e.g., one or any combination of a pulse sensor, an EDA sensor, a body temperature sensor, etc. The motion sensor may include at least one of an accelerometer, a gyroscope, a magnetometer sensor or the like. The motion sensor may be configured to detect motion data of the user, and the motion data of the user may be used for determining, solely or together with other sensor data, an activity type of the user such as exercising, resting, sleeping, or the like, analyses of the activity the user is participated in or the like. The pulse sensor may include a photoplethysmography (PPG) sensor, an ECG sensor, or the like. The pulse sensor may be configured to detect pulse data of the user, and the pulse data may be used for determining, solely or together with other sensor data, a heart rate, HRV, a stress level, an activity type, a body temperature, a respiration rate, or the like, of the user. The body temperature sensor may be configured to obtain skin temperature data and/or core temperature data of the user, where the skin temperature data may be used for predicting a core body temperature of the user. The device 100 may also include other types of sensors, and no limitation is set herein.

In some embodiments, the device 100 may also include one or more sensors and processing tools for detecting, collecting, processing, or displaying one or more environmental parameters captured by the device 100 in a vicinity of the individual.

The environmental parameters can include, for example, one or any combination of positioning information, location, altitude, environmental temperature, environmental humidity, environmental light, weather, air pollution index such as PM2.5 particulate matter content, CO2 content, CO content, which can be captured by, for example, one or more environmental sensors of the device 100. In some examples, the environmental parameters can also include motion data such as motion tracks from at least one of a GPS sensor, a motion sensor (e.g., one or more of an accelerometer, gyroscope, magnetometer, etc.), or a barometer to record additional measurement data such as altitude. In some examples, the environmental parameters can include at least one of: altitude, GPS location, ambient temperature, ambient humidity, ambient light, ambient noise index, an environmental pollution index, or other environmental parameters in the vicinity of the individual that can be captured by the wearable device.

The device 100 may further include one or more communication units. The one or more communication units may be configured to communicate with other devices such as a personal device of the user (such as a handheld device, a smart phone, a tablet, a laptop computer, a desktop computer, a personal digital assistant (PDA), an in-vehicle smart device, another wearable device, a smart home device, or the like) or a server (such as a cloud-based server). The communications can be transmitted wirelessly (e.g., via Bluetooth, RF signal, Wi-Fi signal, near field communications, cellular communications, etc.) or through one or more electrical connections. In some examples, any analog information collected or analyzed during communication can be translated to digital information for reducing the size of information transfers between devices or units.

In an example as shown in FIG. 1, the device 100 can include a sensor unit 155 including at least one of, but not limited to, an image sensor such as a camera, one or more physiological sensors such as a PPG, ECG, EEG, EOG, EMG, EDA sensor, a respiration sensor, a heart rate sensor, a blood pressure sensor, a blood oxygen sensor, and/or a body temperature sensor, at least one of which may include one or more optical detectors 160 and one or more light sources 165, one or more contact pressure/tonometry sensors 170, or one or more motion sensors such as at least one of gyroscopes, magnetometers, or accelerometers 175. These sensors are only illustrative of the possibilities, however, and additional or alternative sensors such as one or more acoustic sensors, electromagnetic sensors, ECG electrodes 120, bio impedance sensors, galvanic skin response sensors, body temperature sensors, or any combination thereof may be included. In some examples, though not depicted in FIG. 1, the device 100 may also include one or more such sensors and/or components on its inside surface (i.e., the surface in contact with the user's tissue or targeted area). It should be understood that the device 100 can be implemented with a different configuration of the sensor unit 155 from what is depicted in FIG. 1 or the examples of the disclosure.

The location of the sensor unit 155 or one or more sensor components of the sensor unit 155 with respect to the user's tissue may be customized to account for differences in body type across a group of users or placement in different locations on a user.

The signal values and additional data collected from the sensor unit 155 may assist a non-transitory computer readable medium or processor in isolating various physiological conditions (e.g., heart rate, oxygen saturation, activity status, body temperature, respiration rate, etc.). The processor may receive data from the sensor unit 155. In some examples, the processor may dynamically filter the data. The processor may analyze the data without regard to a position of the device relative to the user or a position of the user. The processor may filter unwanted signals and isolate only desired signals. For example, the processor may learn which signals are of interest (e.g., which signals are associated with physiological conditions of interest, which may form a basis to determine readiness of a user) and the process may analyze only those signals of interest. In some examples, the processor may be in communication with or include a non-transitory computer-readable medium.

In some embodiments, the sensor unit 155 can be configured to continuously collect data from a user. However, in some examples, certain techniques can be employed to reduce power consumption and conserve battery life of the device 100. For example, while the PPG sensor can be used to continuously monitor blood flow signals of the user, the ECG electrodes 120 can be used periodically or intermittently to collect potentially more accurate blood flow information which can be used to supplement or calibrate the PPG measurements collected and analyzed by the processor.

For example, when the data from one or more accelerometers or gyroscopic components of the device 100 indicates that a user is still or at rest, one or more sensors of the device 100, such as the PPG sensor, which consumes more power than the one or more accelerometers or gyroscopic components, may be turned off to conserve power consumption. However, in some examples, when the data from the one or more accelerometers and/or gyroscopic components of the device 100 indicates that the user is exercising, the one or more sensors of the device 100, such as the PPG sensor, may be turned on to measure the heart rate and/or other physiological parameters of the user. In another example, when the data from one or more accelerometers and/or gyroscopic components of the device 100 indicates that a user is sleeping and the sleep analysis function is turned on, the one or more sensors, such as the PPG sensor, may still need to be turned on even though the data from the one or more accelerometers and/or gyroscopic components of the device 100 indicates that movement of the user is minimal during sleep.

In some examples, the device 100 may also include an input and/or output unit, such as one or any combination of a display unit (not shown), sound unit, tactile unit, or the like, for communicating information to the user (e.g., the wearer of the wearable device) or receiving information from the user. The display unit may be configured to display the images or videos captured by the sensors such as the image sensor, notifications, or alerts.

In some embodiments, the display unit may include an LED indicator having a plurality of LEDs, each emitting light with a different color. The LED indicator can be configured to illuminate in different colors depending on the information being conveyed. For example, where the device 100 is configured to monitor the user's heart rate, the display unit may illuminate light of a first color when the user's heart rate is in a first numerical range, illuminate light of a second color when the user's heart rate is in a second numerical range, and illuminate light of a third color when the user's heart rate is in a third numerical range. In this manner, a user may be able to detect his or her approximate heart rate at a glance, even when numerical heart rate information is not displayed at the display unit, and/or the user only sees the device 100 through the user's peripheral vision (e.g., while exercising).

In some embodiments, the display unit may include a display screen for displaying images, characters, graphs, waveforms, or a combination thereof to the user or a medical professional. By way of example, the device 100 may be configured to detect one or more physiological conditions of the user and provide a readiness score of the user (e.g., determine readiness of the user to partake and/or complete a given activity based on the one or more physiological conditions detected), whereby information displayed on the display screen may be or may include the readiness score. In some examples, the display unit may further include one or more buttons or switches implemented in hardware or software form and configured to receive input by the user. Similarly, in some examples, the display screen may be a touch screen configured to receive input by the user. In some examples, the display unit may also switch or be toggled between displaying information.

In some embodiments, the physiological or environmental information discussed above may be graphically displayed or represented on a display (not shown) of the device 100. The graphical display may be provided to the user as an output. The output may include physiological or environmental information of a user. For example, the information collected may be categorized and then graphically represented as one or more outputs. In some examples, the output may include alert, guidance, or suggestion to the user. In other examples, the output may also include education information pertaining to topics of interest for the user.

In some embodiments, the device 100 may include a housing and a wristband coupled to the housing. The wristband is configured to secure the device 100 to the user's body. The housing is configured to accommodate or accept other components of the device 100, such as a sensor unit, a communication unit, an input and/or output unit, a processing unit, a storage unit, or any combination thereof. In some examples, the device 100 may include a back shell that comes into contact with the user's skin when the device 100 is worn by the user. The back shell may include a light-transmitting configuration and a light-blocking configuration. The light-transmitting configuration may, for example, include a light-transmitting back shell, or one or more openings provided in a non-light-transmitting back shell with light-transmitting lenses disposed in the openings. The light-blocking configuration may include, for example, a light-blocking bracket, a light-blocking ink layer, a light-blocking foam, or any combination thereof. At least one sensor in the sensor unit, such as a PPG sensor, may transmit light signals to the user's skin through the back shell and receive light signals returned from the user's skin through the back shell.

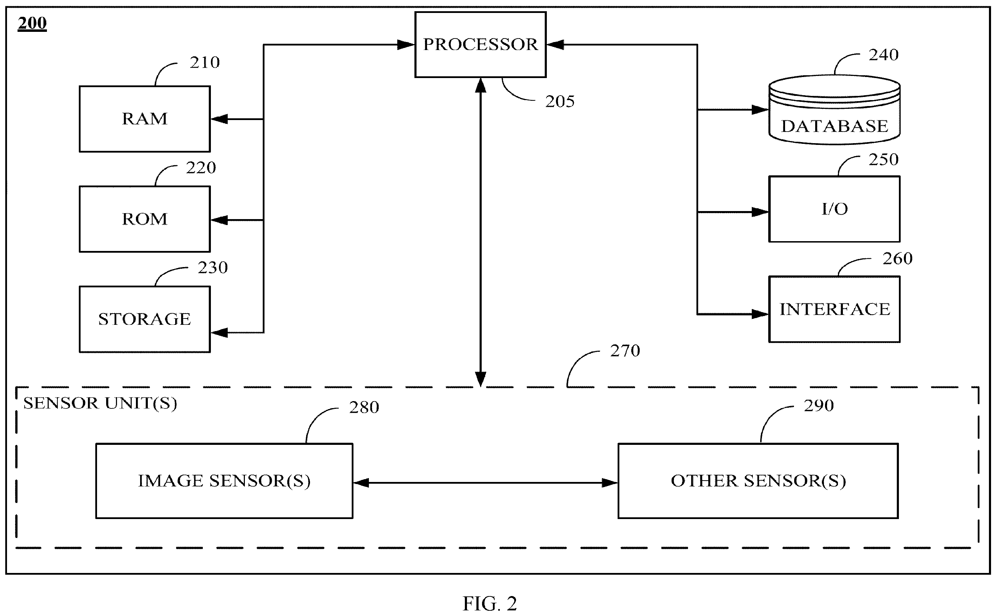

FIG. 2 depicts an example of a computing device 200 that may be used with or incorporated into a wearable device. The computing device 200 is representative of the type of computing device that may be present in or used in conjunction with at least some aspects of the device 100, or any other device including electronic circuitry. For example, the computing device 200 may be used in conjunction with any one or more of transmitting signals to and from the one or more optical sensors or acoustical sensors, sensing or detecting signals received by one or more sensors of the device 100, processing received signals from one or more components or units of the device 100 or a secondary device, and storing, transmitting, or displaying information. The computing device 200 may be or may be included within the device 100. The computing device 200 may be a mobile terminal or remote device that is in communication with the device 100. The computing device 200, the device 100, or both may be in communication with a server (e.g., a cloud-based server). For example, the computing device 200 may be a separate device (e.g., a mobile terminal) from the device 100, and both the computing device 200 and the device 100 may be in direct communication with the server. Alternatively, the computing device 200 may be in direct communication with the server and the device 100 may be in communication with the server via the computing device 200. It should also be noted that the computing device 200 is illustrative only and does not exclude the possibility of another processor- or controller-based system being used in or with any of the aforementioned aspects of the device 100.

In some examples, the computing device 200 may include one or more hardware and/or software components configured to execute software programs, such as software for obtaining, storing, processing, and analyzing signals, data, or both. For example, the computing device 200 may include one or more hardware components such as, for example, a processor 205, a random-access memory (RAM) 210, a read-only memory (ROM) 220, a storage 230, a database 240, one or more input/output (I/O) units 250, an interface 260, and a sensor unit 270.

Alternatively, and/or additionally, the computing device 200 may include one or more software components such as, for example, a computer-readable medium including computer-executable instructions for performing techniques or implement functions of tools consistent with certain disclosed embodiments. It is contemplated that one or more of the hardware components listed above may be implemented using software. For example, the storage 230 may include a software partition associated with one or more other hardware components of the computing device 200. The computing device 200 may include additional, fewer, and/or different components than those listed above. It is understood that the components listed above are illustrative only and not intended to be limiting or exclude suitable alternatives or additional components.

The processor 205 may include one or more processors, each configured to execute instructions and process data to perform one or more functions associated with the computing device 200. The term "processor," as generally used herein, refers to any logic processing unit, such as one or more central processing units (CPUs), digital signal processors (DSPs), application specific integrated circuits (ASICs), field programmable gate arrays (FPGAs), and similar devices. In an example as illustrated in FIG. 2, the processor 205 may be communicatively coupled to the RAM 210, the ROM 220, the storage 230, the database 240, the I/O unit 250, the interface 260, and the sensor unit 270. The processor 205 may be configured to execute sequences of computer program instructions to perform various processes, which will be described in detail below. The computer program instructions may be loaded into the RAM 210 for execution by the processor 205.

The RAM 210 and the ROM 220 may each include one or more devices for storing information associated with an operation of the computing device 200 and/or the processor 205. For example, the ROM 220, may include at least one memory device configured to access and store information associated with the computing device 200, including information for identifying, initializing, and monitoring the operation of one or more components and subsystems of the computing device 200. The RAM 210 may include at least one memory device configured to storing data associated with one or more operations of the processor 205. For example, the ROM 220 may load instructions into the RAM 210 for execution by the processor 205.

The storage 230 may include any type of storage device configured to store information that the processor 205 may use to perform processes consistent with the disclosed embodiments.

The database 240 may include one or more software and/or hardware components that cooperate to store, organize, filter, and/or arrange data used by the computing device 200 and/or the processor 205. For example, the database 240 may include user profile information, historical activity and user-specific information (e.g., historical information pertaining to the mental health of a specific user), physiological parameter information, predetermined menu/display options, and other user preferences. Alternatively, the database 240 may store additional and/or different information. For example, the database 240 may include information to establish a machine learning model such as a large language model (LLM) that can receive inputs from the I/O unit 250 or the sensor unit 270.

The I/O unit 250 may include one or more components configured to communicate information with a user associated with the computing device 200. For example, the I/O unit 250 may include one or more buttons, switches, or touchscreens to allow a user to input parameters associated with the computing device 200. The I/O unit 250 may also include a display including a graphical user interface (GUI) and/or one or more light sources for outputting information to the user. The I/O unit 250 may also include one or more communication channels for connecting the computing device 200 to one or more secondary or peripheral devices such as, for example, a desktop computer, a laptop, a tablet, a smart phone, a flash drive, or a printer, to allow a user to input data to or output data from the computing device 200.

The interface 260 may include one or more components configured to transmit and receive data via a communication network, such as the internet, a local area network, a workstation peer-to-peer network, a direct link network, a wireless network, or any other suitable communication channel. For example, the interface 260 may include one or more modulators, demodulators, multiplexers, demultiplexers, network communication devices, wireless devices, antennas, modems, and any other type of device configured to enable data communication via a communication network.

The computing device 200 may further include the sensor unit 270. In some examples, the sensor unit 270 may include an image sensor 280, and/or other sensors 290 such as an accelerometer, an optical sensor, an acoustical sensor, an ambient light sensor, a pressure sensor, a contact sensor, an electromagnet sensor, an ECG sensor, an EEG sensor, an EOG sensor, an EMG sensor, an EDA sensor, a bio impedance sensor, etc. It should be noted that these sensors are only illustrative of a few possibilities and the sensor unit 270 may include alternative or additional sensors suitable for use in the device 100. It should also be noted that although one or more sensors are described collectively as the sensor unit 270, any one or more sensors within the device 100 may operate independently of any one or more other sensors. Moreover, in addition to collecting, transmitting, and receiving signals or information to and from the sensor unit 270 at the processor 205, any of the one or more sensors of the sensor unit 270 may also be configured to collect, transmit, or receive signals or information to and from other components or units of the computing device 200, including but not limited to the database 240, the I/O unit 250, or the interface 260.

As described above with respect to FIG. 1, the accelerometer can be used to detect large-scale motions of a subject indicative of physical activity (e.g., strength training, running, walking swimming, etc.) The same accelerometer can be used to determine the onset of a sleep period through the detection of a lack of motion. The acoustical sensor and/or optical sensor can be used to detect and/or monitor heart rate. However, in case the sensitivity of the acoustical sensor and/or optical sensor that detect heart rate is not enough to detect relatively slow heart rate during sleeping, in some examples, upon determining that the subject is engaged in sleep, the sensitivity of the acoustical sensor and/or optical sensor can be reconfigured to detect a significantly lower heart rate. Alternatively, one or more acoustical sensors and/or optical sensors can be dedicated to, and configured for, detecting relatively slow heart rate during sleeping while one or more other acoustical sensors and/or optical sensors are used to detect regular heart rate during physical activity.

As described above, a person skilled in the art will note that all or a portion of the aspects of the disclosure described herein can be implemented using a general-purpose computer/processor with a computer program that, when executed, carries out any of the respective techniques, algorithms, and/or instructions described herein. In addition, or alternatively, for example, a special-purpose computer/processor, which can contain specialized hardware for carrying out any of the techniques, algorithms, or instructions described herein, can be utilized.

Similarly, all or a portion of the aspects of the disclosure described herein can be implemented by the device 100 (e.g., by the processor 205 when the computing device 200 is incorporated into the device 100), by a server in communication with the device 100 and/or the computing device 200, or both. Additionally, all or a portion of the aspects of the disclosure described herein (e.g., steps, procedures, processes, etc.) may be performed by the device 100 or a secondary companion device (e.g., a mobile terminal, a client device, other remote device, etc.). For example, a portion of the steps or procedures described herein may be performed by the aforementioned server while another portion of the steps or procedures may be performed by the secondary companion device.

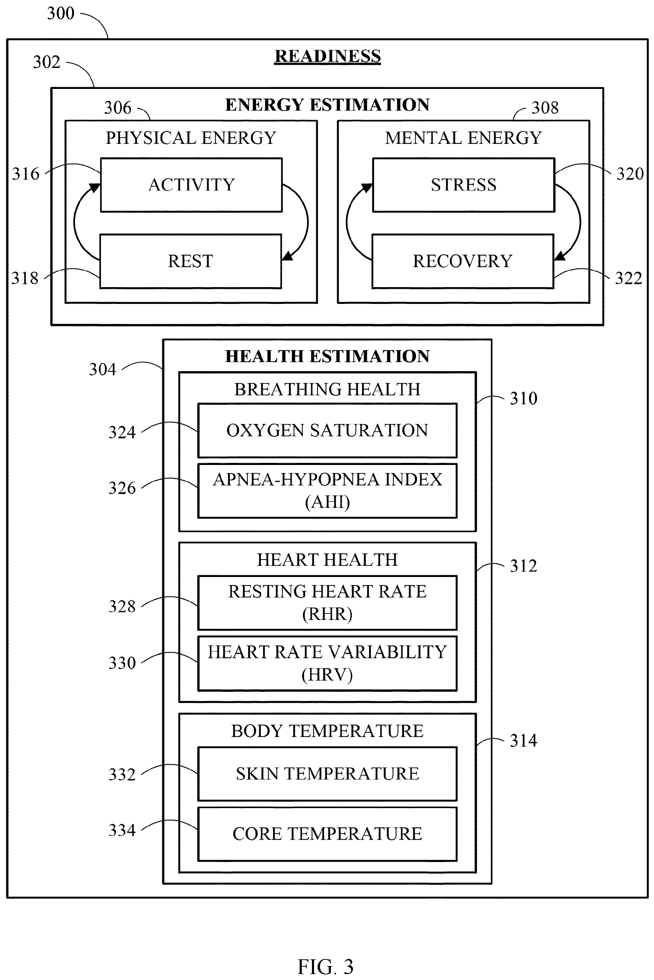

FIG. 3 is a schematic block diagram depicting an example of components that may be utilized to determine a readiness 300 of a user in accordance with the present teachings. As discussed above, the readiness 300 of the user may be determined and/or provided as a readiness score of the user, which may be correlated to, associated with, or an indication of the user's physical and/or mental ability to partake in and/or complete a given activity. The activity may be any physical activity (e.g., exercise, physical chores, physical tasks, etc.), mental activity (e.g., work-related tasks, reading, other mental tasks, etc.), or a combination thereof. It should be noted that the components described herein with respect to FIG. 3 are intended as illustrative examples of the components of the readiness only and that additional or alternative components of the readiness may also be implemented in accordance with the present teachings.

As discussed further herein, the readiness 300 of the user (e.g., the readiness score of the user) may be or may provide an indicator of the user's physical ability and/or mental capacity at a given point in time. That is, the readiness 300 of the user may provide personalized, real-time information to the user based on the physiological functioning and health-related behaviors of the user (e.g., exercise, rest, sleep, etc.) While the readiness 300 of the user may be determined and provided to the user as a readiness score, additional information associated with the readiness 300 of the user may also be provided to the user. For example, the additional information may include guidance (e.g., instructions, recommendations, or information associated with the readiness 300 of the user determined), information associated with one or more of the components utilized to determine the readiness 300 of the user (e.g., one or more of the components shown in FIG. 3 and discuss in further detail below), or both.

Turning back to FIG. 3, the readiness 300 of the user (e.g., the readiness score) may be determined based on one or more components, whereby the one or more components may be considered sub-components of the readiness 300 of the user. By way of example, the one or more components may each be or may each include a value, and the values of the one or more components may be utilized to determine the readiness score of the user. As such, the one or more components of the readiness 300 of the user may be parameter values cumulatively tracked and evaluated to determine the readiness score.

As shown in FIG. 3, the readiness 300 of the user may be determined based on an energy estimation component 302 and a health estimation component 304. In some embodiments, the energy estimation component 302 may be or may include indicators of energy of the user for physical energy 306 and mental energy 308. The physical energy 306 may be used interchangeably with physical recovery, or refer to, or be associated with, physical energy depletion (i.e., consumption) of the user and/or physical energy repletion (i.e., recovery) of the user. Similarly, the mental energy 308 may be used interchangeably with mental recovery, or refer to, or be associated with, mental energy depletion of the user and/or mental energy repletion of the user. Thus, physical energy 306 and mental energy 308 of the user as described herein may also refer to overall recovery or the energy depletion and/or energy repletion unless otherwise indicated. For example, physical energy 306 may refer to physical recovery or physical energy recovery of the user and mental energy 308 may refer to mental recovery or mental energy recovery of the user.

In some embodiments, the health estimation component 304 may be or may include indicators of health for at least one of breathing health 310, heart health 312, and body temperature 314. In some examples, the physical energy 306, the mental energy 308, the breathing health 310, the heart health 312, and the body temperature 314 of user may collectively indicate the readiness 300 of the user. That is, the components 306-314 may, when taken as a whole, indicate whether the user is ready for a particular activity or needs additional rest and recovery before such an activity can be done successfully.

In some embodiments, the components 306-314 may be determined based on data obtained in any desired manner. By way of example, the components 306-314 may be determined based on physiological signals (or sensor signals) detected by the device 100 when the device 100 is worn by the user. The physiological signals detected by the device 100 may be associated with one or more physiological conditions of the user, whereby the one or more physiological conditions of the user may be associated with one or more of the components 306-314.

In addition to physiological signals detected by the device 100, one or more of the components 306-314 may also, in certain cases, be partially or entirely based on subjective feedback provided by the user. For example, the user, via the device 100 or another device, may provide feedback with respect to their subjective feeling of readiness. The feedback may be in the form of answers to a questionnaire and/or other data manually input by the user. Such feedback may then be utilized as an additional or alternative metric to those determined based on the physiological signals detected by the device 100. That is, the readiness 300 (e.g., the readiness score) of the user may be determined based on objective data (e.g., the physiological signals detected by the device 100), subjective data (e.g., the feedback from the user), or both. As a result, the readiness 300 (e.g., the readiness score) may be personalized and more tailored to the user.

By way of example, the readiness 300 (e.g., the readiness score) of the user may be based upon both the objective data (e.g., the physiological signals detected by the device 100) and the subjective feedback (e.g., answers to questions provided by the user) such that the determined readiness score and/or one or more determined readiness component scores (e.g., one or more scores associated with the components 306-314) may be adjusted based upon the user's subjective experience. That is, the user may be prompted to provide answers to questions specifically tailored to one or more of the components 306-314 to input how the user subjectively feels with respect to the one or more components 306-314. For example, the user may provide feedback via the device 100 to input how much physical energy 306 and/or mental energy 308 the user's currently feels that they have. Based on such inputs, the determination (e.g., calculation or estimation) of one or more of the components 306-314 based on the objective data (e.g., the physiological signals detected) may be modified or otherwise adjusted to take into account the user's input.

For example, the physical energy 306 estimated using objective data may indicate the user currently has a higher level of physical energy. However, the user may provide feedback that they are currently feeling physically tired. As a result, a readiness component score associated with the physical energy 306 of the user may be adjusted (e.g., decreased) based upon the user's feedback. Similarly, or additionally, the readiness 300 (e.g., the readiness score) may be adjusted to account for the dissimilarities between the objectively determined physical energy 306 of the user and the subjectively input physical energy 306 of the user. It should be noted that a similar process may be completed for any of the components 306-314 discussed herein.

In some embodiments, the readiness 300 of the user may be determined based on the physical energy 306 of the user. As shown in FIG. 3, physical energy 306 may be determined based on periods of activity 316 and rest 318. The activity 316 and the rest 318 of the user may be determined based on one or more of the physiological conditions of the user at a given point in time, such as heart rate, blood pressure, body temperature, or other physiological conditions. For example, a period of activity 316 of the user or a period of rest 318 of the user may be determined at least in part by the aforementioned physiological conditions. The periods of activity 316 and rest 318 of the user may indicate levels (e.g., durations) of physical exertion and physical rest such that the physical energy 306 of the user may be determined. That is, physical energy of the user may be determined based on a balance between the activity 316 of the user and the rest 318 of the user.

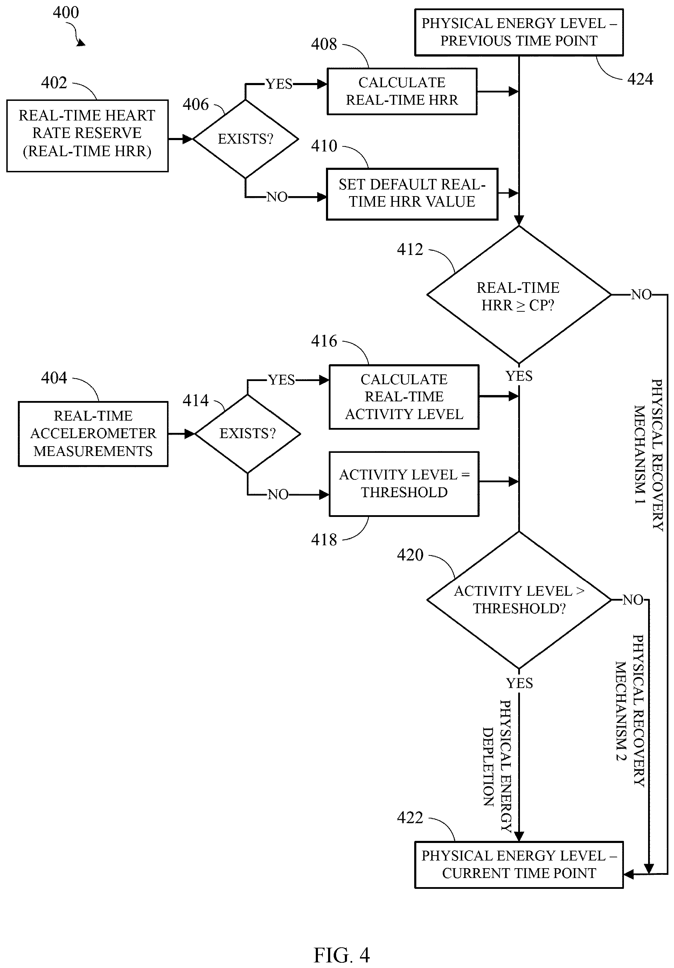

In some examples, the physical energy may be determined based on at least one of heart rate data obtained from the heart rate sensor and motion data obtained from the motion sensor. For example, the physical energy of the user is determined based on one or more activity metrics and/or one or more heart rate metrics of the user. By way of example, and as discussed in further detail below, the physical energy 306 may be determined at least partially based on the critical point model that depends on exercise intensity and physical energy reserve (e.g., physical energy level or physical energy value at the previous time point). That is, the physical energy 306 may take into account an intensity of the activity 316 (e.g., the exercise). For example, it may be determined that the physical energy of the user has been consumed when the intensity of the activity 316 (e.g., high, medium, low) is higher than a critical point (e.g., a predefined threshold value for one or more parameters used to determine the intensity of the activity 316). Conversely, it may be determined that the physical recovery 306 of the user is taking place when the intensity of the activity 316 is lower than the critical point (e.g., when the physical energy of the user consumed for the activity 316 is lower than the critical point). In other words, it may be determined whether, based on the periods of activity 316 and periods of rest 318, the user is in fact physically recovering or instead expending physical energy (i.e., physical energy consumption). A method of determining the physical energy 306 of the user is discussed in further detail below with respect to FIG. 4.

In some embodiments, the readiness 300 of the user may also be determined based on the mental energy 308 of the user. As shown in FIG. 3, mental energy 308 may be determined based on periods of stress 320 and recovery 322. The stress 320 and the recovery 322 of the user may be based on one or more of the physiological conditions of the user at a given point in time, such as heart rate, blood pressure, body temperature, or other physiological conditions. For example, a period of stress 320 of the user or a period of recovery 322 of the user may be determined at least in part by the aforementioned physiological conditions. The periods of stress 320 and recovery 322 may indicate levels (e.g., durations) of mental exertion and mental recovery (e.g., mental rest based upon a lack of activity and/or sleep) such that the mental energy 308 of the user may be determined. That is, the mental energy 308 may be determined based on a balance between the stress 320 of the user and the recovery 322 of the user.

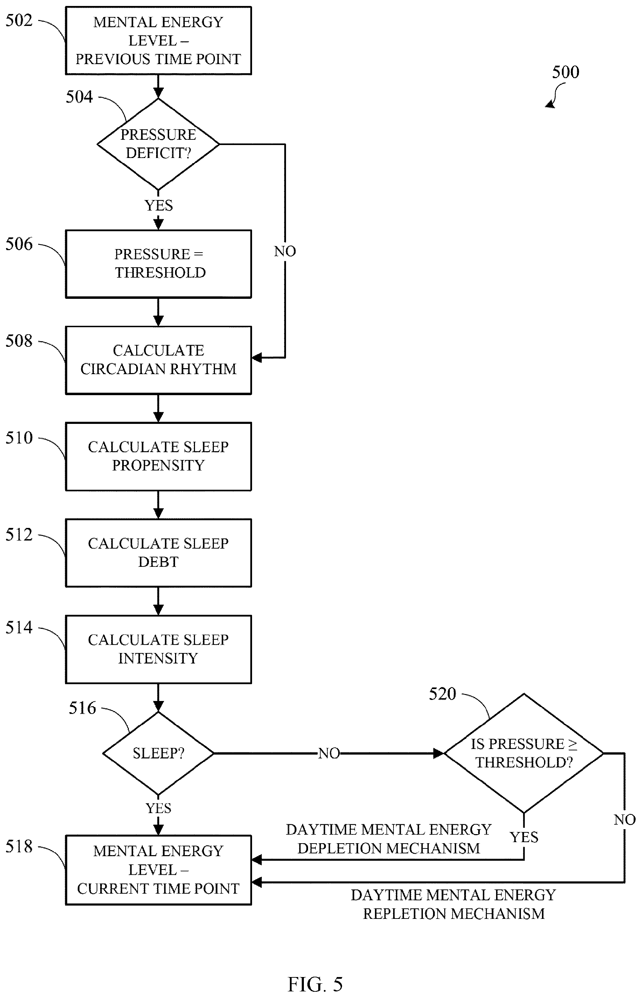

By way of example, and as discussed in further detail below, the mental energy 308 may be determined at least partially based on the sleep, activity, fatigue, and task effectiveness (SAFTE) model. The SAFTE model may be implemented herein to effectively predict the effects of sleep metrics, circadian rhythms, and cognitive load on mental fatigue (e.g., stress 320) and mental recovery (e.g., recovery 322). That is, a method of determining the mental energy 308 herein may be at least partially based on the SAFTE model and may incorporate various metrics associated with the user, such as but not limited to, circadian rhythm factors and various sleep metrics (e.g., sleep duration, start and end times of a sleep cycle, interruptions in sleep, sleep quality, etc.) Such metrics associated with the user may be determined at least in part by physiological signals detected by the device 100 or other devices monitoring the user. By utilizing the SAFTE model and various other metrics, the method of determining the mental energy 308 may dynamically determine periods of sleep of the user as well as periods of quiet immobility (e.g., meditation) of the user, which may both contribute to recovery of the mental energy 308 of the user. That is, it may be determined whether, based on the periods of stress 320 and periods of recovery 322, the user is in fact mentally recovering or instead expending mental energy (i.e., mental energy consumption). A method of determining the mental energy 308 of the user is discussed in further detail below with respect to FIG. 5.

In some embodiments, the readiness 300 (e.g., the readiness score) of the user may also be determined based on the health 304 of the user, which may include the breathing health 310, the heart health 312, and the body temperature 314 of the user. Each of these components 310-314 may be determined based on one or more parameters. The one or more parameters of each of the components 310-314 may be, or may be determined based on, one or more physiological signals captured by the device 100 when worn by the user or another device monitoring the user. That is, the components 310-314 may be determined based at least partially on the one or more physiological conditions of the user, whereby such physiological conditions of the user may be determined based on the one or more physiological signals.

The breathing health 310 of the user may be associated with breathing of the user and the amount of oxygen in the user's blood. Poor breathing may be an indication of a health issue of the user. By way of example, if the user is sick, it may be difficult for the user to breathe, which may in turn cause the oxygen in the user's blood to drop. Additionally, if the user exhibits sleep disordered breathing (e.g., obstructive sleep apnea), pauses in breathing during sleep may cause the oxygen in the user's blood to drop frequently during the sleep. Such poor breathing, especially during sleep, may hinder the user's ability to recover physically and/or mentally. Thus, the breathing health 310 may be utilized in conjunction with the physical energy 306 and/or the mental energy 308 determined to more accurately track whether the user physically and/or mentally recovers. Similarly, the breathing health 310 may be utilized to modify the determinations made for the physical energy 306 and/or the mental energy 308 to more accurately reflect any breathing health conditions exhibited by the user.

In some embodiments, the breathing health 310 of the user may be determined (e.g., measured or estimated) based on fluctuations/changes in oxygen saturation 324 and/or the apnea-hypopnea index (AHI) 326. For example, fluctuations in oxygen saturation 324 and/or the AHI 326 may be determined (e.g., extracted) from a photopolythesogram (PPG) signal detected by the device 100 when worn by the user. Using domain knowledge boundaries (e.g., predefined boundaries for values of oxygen saturation 324 and/or the AHI 326), a level of oxygen saturation 324 and AHI 326 may be determined. It should be noted that the above methodology to determine the breathing health 310 is intended an example and other methods of determining the breathing health 310 of the user may be possible.

The heart health 312 of the user may be associated with functionality of the heart of the user. For example, the heart health 312 may be associated with a heart rate of a user, whereby the heart rate may be an indicator of how fast or slow the user's heart is beating. The heart rate and variability thereof may be indicators of how healthy the user's heart may be and how well the user's body may adapt to physical and/or mental changes. Thus, the heart health 312 may be utilized in conjunction with the physical energy 306 and/or the mental energy 308 determined to more accurately track whether the user physically and/or mentally recovers. Similarly, the heart health 312 may be utilized to modify the determinations made for the physical energy 306 and/or the mental energy 308 to more accurately reflect any heart conditions exhibited by the user.

In some examples, the heart health 312 of the user may be determined (e.g., measured or estimated) based on a resting heart rate (RHR) 328 and/or heart rate variability (HRV) 330 of the user. The RHR 328 may measure or be a measurement of the heart rate of the use while at rest. A lower RHR 328 may be an indication of better heart health of the user while a higher RHR 328 may indicate poorer heart health. HRV 330 may measure or be a measurement of how well the user's body (including the heart of the user) can adapt to physical and/or mental changes (e.g., physical and/or mental exertion or stress). A higher HRV 330 may be an indication of less stress and better overall health of the user while a lower HRV 330 may indicate higher stress and poorer over health of the user. Using domain knowledge boundaries (e.g., predefined boundaries for values of the RHR 328 and/or HRV 330), the RHR 328 and HRV 330 may be determined. It should be noted that other factors other than the RHR 328 and HRV 330 of the user may be utilized to determine the heart health 312 of the user.

The body temperature 314 of the user fluctuates (e.g., increases and/or decreases) throughout the course of the day based upon (e.g., in response to) the environment, meals, exercise, and other factors experienced by the user. For example, the user's brain may regulate their body temperate in response to any conditions. A body temperature that is high than normal may indicate oncoming illness or significant stress for the user, suggesting that the user may need additional time to rest and/or recover during the day.

In some embodiments, the body temperature 314 of the user may be determined (e.g., measured or estimated) based on a skin temperature 332 of the user and/or a core temperature 334 (i.e., core body temperature) of the user. For example, fluctuations in the core temperature 334 may be estimated based on measuring the skin temperature 332 of the user, such as during sleep, and the core temperature 334 may be determined based on the skin temperature measurement and associated heart rate measurement. If the fluctuation of the core temperature 334 estimates exceeds about 1° C or another predefined threshold, it may indicate a potential deviation from a normal temperature range of the user. Such deviation may be a sign of oncoming illness or significant stress.

FIG. 4 illustrates a schematic flow diagram of an example of a method 400 for dynamically determining the physical energy 306 of the user. As discussed above, the physical energy 306 may be determined based on periods of activity 316 and periods of rest 318 of the user, whereby the user may alternate between the periods of activity 316 and the periods of rest 318.The physical energy 306 may also be based on, or take into account, an intensity (e.g., low, moderate, high) of the activity since different levels of intensity of a particular activity may consume different amounts of the user's physical energy, thereby depleting the overall physical capacity (e.g., the readiness) of the user.

By way of example, the physical energy 306 may be determined (e.g., calculated) by using the critical point model based on heart rate data and/or activity intensity and physical energy reserve. Physical energy consumption may occur when the heart rate and/or intensity of the activity is higher than a critical point (e.g., a predefined threshold), whereas physical energy recovery may occur when the physical energy consumption is lower than the critical point. Such determinations may be made based on the exercise power of the user and/or data associated with the activity level of the user (e.g., data obtained by an accelerometer or other sensor of the device 100 when worn by the user).

In some embodiments, the real-time exercise intensity or physiological exercise power of the user may be determined and based at least partially upon heart rate data (such as a real-time heart rate reserve (HRR) 402) of the user and motion data 404 (e.g., accelerometer data obtained by the device 100 or another portable device of the user). The real-time HRR of the user may be utilized to assess the physical fitness (e.g., the cardiovascular fitness) of the user to determine the user's real-time exercise intensity or physiological exercise power based upon a particular activity (e.g., based upon a particular activity intensity). The real-time exercise intensity or physiological exercise power of the user may then be used to assess the activity level of the user and thereby determine the physical energy 306 (or physical depletion) of the user.

It should also be noted that additional metrics may also be utilized to determine real-time exercise intensity or physiological exercise power of the user to thereby assess the activity level of the user and determine the physical energy 306 of the user. By way of example, in lieu of, or in addition to, real-time HRR, the present teachings may also contemplate determining the real-time heart rate recovery and/or real-time heart rate intensity of the user. Thus, the real-time HRR of the user as described herein is only intended as an example metric for determining the physical energy 306.

As shown in FIG. 4, the real-time heart rate data of the user may be monitored at 406 to determine if the real-time HRR 402 exists (i.e., is present) at that particular point in time. The real-time HRR may be or may be associated with at least one of real-time heart rate, resting heart rate, maximum heart rate, or heart rate reserve (HRR) (e.g., a difference between the maximum heart rate and the resting heart rate of the user) of the user. At 406, the method 400 determines whether the real-time HRR 402 is present - that is, whether calculating the real-time HRR 402 may be possible for the user based upon the current activity and/or status of the user.

In some examples, if it is determined that the real-time heart rate data exists at 406, the real-time HRR 402 may be determined (e.g., calculated or estimated) at 408 for a time point (t) based upon at least one of a resting heart rate of the user, a max heart rate of the user, and a current heart rate of the user (e.g., a real-time heart rate of the user). For example, the real-time HRR 402 may be determined based on a difference between the current heart rate of the user and the max heart rate of the user, a difference between the current heart rate of the user and the resting heart rate of the user, a difference between the max heart rate and the resting heart rate of the user, or any combination thereof. It should be noted that the user's real-time heart rate and/or the resting heart rate of the user may be determined (e.g., measured or estimated) based on one or more physiological signals detected by the device 100 or another portable device of the user. Additionally, the max heart rate of the user may be estimated based upon the user's personal data such as age (which may be manually input by the user via the device 100 or another portable device).

In other examples, if it is determined that the real-time heart rate data does not exist at 406 (e.g., the real-time HRR 402 is not able to be calculated based upon the current conditions), a default real-time HRR value may be set at 410. By way of example, the default real-time HRR value set at 410 may be 15. However, any default real-time HRR value may be possible using the method 400. Alternatively, the real-time HRR value at the current time point may be deduced by using an algorithm based on the history HRR data of the user, the real-time HRR values at other time points close to the current time point or the like for example.

Once the real-time HRR value is calculated at 408 or the default real-time HRR value is set at 410, the value is then compared to the predefined critical point (CP) at 412. The CP may be any predefined threshold value that is compared to the real-time HRR value - either calculated at 408 or set at 410 - to determine whether the real-time HRR value is greater than or equal to the CP. The CP may be the same as or different from the default real-time HRR value set at 410. For example, the CP may also be 15. Thus, the CP may be considered a threshold real-time HRR value. In some examples, the CP may be a customized value for the user and may be determined based at least partially on the user's personal data, history physiological data of the user or the like.

If the real-time HRR value is determined at 412 to be greater than or equal to the CP, the activity level (e.g., the activity 316) may be evaluated. In some examples, the real-time accelerometer measurements 404 may be used to evaluate the activity level (e.g., low, intermediate, high) of the user. For example, it may be first be determined at 414 whether real-time motion data such as real-time accelerometer measurements 404 exists.

If the real-time motion data such as the real-time accelerometer measurements exists, a real-time activity level may be calculated at 416 based upon the real-time accelerometer measurements. For example, the real-time activity level may be calculated at 416 based on at least one of a speed of movement of the user, an amount of movement of the user, a duration of time of movement of the user, one or more physiological conditions of the user (e.g., heart rate, blood pressure, breathing conditions, body temperature of the user, etc.), or one or more physiological signals detected by the device 100 or another portable device of the user. In some examples, the determination at 416 may be compared to one or more ranges of activity level (e.g., low, intermediate, high) to categorize the activity level accurately.

If the real-time motion data such as the real-time accelerometer measurements 404 does not exist at 414, the activity level may set at a value equal to a predefined threshold at 418. For example, the predefined threshold may be a level of the activity that is sufficient for the user to exert physical energy such that physical recovery is not possible. For another example, the predefined threshold may be categorized as a low activity level, whereby if the activity level is low, the user may be active yet not exerting excessive physical energy such that at least some physical recovery is possible (though not as much physical recovery as if the user was not partaking in any activity (e.g., resting)). In some examples, the activity level or the predefined threshold of the activity level may be deduced based on history motion data, history activity level data of the user or the like.

Once the activity level is calculated at 416 or set equal to the threshold at 418, the activity level is then compared to the threshold at 420 to determine whether the activity level is greater than the threshold. In some examples, using the critical point model, multiple hypotheses may exist to determine whether the user is in a state of physical energy recovery or physical energy depletion based upon comparing the activity level to the threshold at 420. In some examples, determining whether the user is in a state of physical energy recovery or physical energy depletion may also be based upon comparing the real-time HRR value to the CP at 412. Such a determination may then be used to determine (e.g., calculate or estimate) the physical energy level of the user at the current time point (t) 422. In some examples, the physical energy level of the user at the current time point (t) 422 may also be compared to the physical energy level of the user at a previous time point (t-1) 424 to determine whether the user is in a state of physical energy recovery or physical energy depletion. It should be noted that the comparison of physical energy levels of the user between the current time point (t) 422 and the previous time point (t-1) 424 may be done before, during, or after determining a rate of physical energy recovery or physical energy depletion.

Illustrative scenarios will now be described with respect to the method 400 shown in FIG. 4, where it is assumed that the physical energy level of the user is determined or updated iteratively on a time-point (e.g., at intervals of one or more minutes or seconds) basis.

In a first scenario, the comparison between the activity level of the user and the threshold may be further determined based upon a first hypothesis of the critical point model where the real-time HRR value of the user is greater than or equal to the CP. Physical energy depletion may occur when the real-time HRR value is greater than or equal to the CP at 412 and the activity level (e.g., exercise intensity) is greater than the threshold at 420, as indicated along the lead line from 412 to 422 in FIG. 4.

In such a scenario, the physical energy level at the current time point (t) at 422 may be determined in accordance with physical energy depletion based upon at least one of the physical energy level of the user at the previous time point (t-1), the real-time HRR at the current time point (t) (e.g., determined as described above), the real-time HRR at the previous time point (t-1) (e.g., determined as described above), the CP, or a physical energy consumption rate of the user. In some examples, the physical energy consumption rate of the user may depend on the heart rate conditions (e.g., based on one or any combination of maximum heart rate, resting heart rate, history heart rate data, etc.) of the user. In some examples, the physical energy consumption rate of the user may be correlated to the amount of time it takes the user to deplete or recover (i.e., recharge) his/her physical energy level based upon a particular activity and/or current circumstances. In some examples, the physical energy consumption rate of the user may be determined based on a difference between the real-time HRR of the user and the CP, e.g., a bigger difference between the real-time HRR of the user and the CP corresponds to a greater physical energy consumption rate of the user, or vice versa. For example, the physical energy consumption rate of the user has a linear dependency relationship with the difference between the user's real-time HRR at the current time point (t) and the CP. In other examples, the physical energy consumption rate of the user is a preset value. In some examples, the physical energy level at the current time point (t) at 422 may be determined based on a difference between the real-time HRR at the current time point (t) or the real-time HRR at the previous time point (t-1) and the CP. In some examples, the physical energy level at the current time point (t) at 422 may be determined based on an accumulation of the physical energy level of the user at the previous time point (t-1) and the physical energy consumption rate of the user. In some examples, the physical energy level at the current time point (t) at 422 may be determined based on the physical energy level of the user at the previous time point (t-1) as well as a physical energy consumption amount of the user, where the physical energy consumption amount of the user may be determined based on the real-time HRR at the current time point (t) or at the previous time point (t-1), and/or determined based on the heart rate conditions of the user. In some examples, the physical energy consumption rate of the user is determined based on the difference between the user's real-time HRR at the current time point (t) or at the previous time point (t-1) and CP, as well as at least one of the user's resting heart rate, maximum heart rate, or historical heart rate.

In a second scenario, it may be determined based upon a second hypothesis of the critical point model that physical energy recovery occurs when the real-time HRR value of the user is less than the CP at 412.

In such a scenario, the physical energy level of the user at the current time point (t) at 422 may be determined in accordance with a first physical recovery mechanism or model (e.g., physical recovery mechanism 1, as indicated along the right line from 412 to 422 in FIG. 4). The physical energy level of the user at the current time point (t) may be determined based upon at least one of the physical energy level of the user at the previous time point (t-1), the real-time HRR at the current time point (t) (e.g., determined as described above), the real-time HRR at the previous time point (t-1) (e.g., determined as described above), or the CP. In some examples, the physical energy level of the user at the current time point (t) at 422 may be determined based on a difference between the real-time HRR of the user at the current time point (t) or at the previous time point (t-1) and the CP. In some examples, the physical energy level of the user at the current time point (t) at 422 may be determined based on the physical energy level of the user at the previous time point (t-1) and a first physical energy recovery rate of the user. For example, the physical energy level of the user at the current time point (t) may be an accumulation (e.g., sum) of the physical energy level of the user at the previous time point (t-1) and the first physical energy recovery rate. In some examples, the first physical energy recovery rate of the user may be determined based on at least one of the user's real-time HRR value, the physical energy level of the user at the previous time point (t-1) or the physiological conditions of the user. For example, the first physical energy recovery rate of the user may be determined based on a difference between the real-time HRR value at the current time point or at the previous time point and the CP. For example, a greater difference between the user's real-time HRR value at the current time point (t) and the CP results in a greater first physical energy recovery rate of the user. For example, the first physical energy recovery rate of the user has an exponential dependency relationship with the difference between the user's real-time HRR value at the current time point (t) and the CP. For another example, the first physical energy recovery rate of the user may be determined based on the physical energy level of the user at the previous time point. For yet another example, a greater physical energy level of the user at the previous time point (t-1) results in a greater first physical energy recovery rate of the user. For yet another example, the first physical energy recovery rate of the user may be determined based on the difference between the real-time HRR of the user at the current time point or at the previous time point and the CP, as well as the physical energy level of the user at the previous time point. In some other examples, the first physical energy recovery rate of the user is a preset value.

In a third scenario, based upon a third hypothesis of the critical point model, physical energy recovery may occur when the real-time HRR value of the user is greater than or equal to the CP at 412 and the activity level is less than the threshold at 420.

In such a scenario, the physical energy level of the user at the current time point (t) at 422 may be determined in accordance with a second physical recovery mechanism or model (e.g., physical recovery mechanism 2, as indicated along the lead line from 420 to 422 in FIG. 4). The physical energy level of the user at the current time point (t) may be determined based upon at least one of the physical energy level of the user at the previous time point (t-1), the real-time HRR at the current time point (t) (e.g., determined as described above), or the real-time HRR at the previous time point (t-1). In some examples, the physical energy level of the user is updated over time in accordance with an exponential function. In some examples, the physical energy level of the user at the current time point (t) may be determined based on an accumulation of the physical energy level of the user at the previous time point (t-1) and a second physical energy recovery rate. In some examples, the physical energy recovery in the third scenario is slower than that in the second scenario, e.g., the second physical energy recovery rate for the third scenario is less than the first physical energy recovery rate in the second scenario. In some examples, the second physical energy recovery rate of the user may be determined based on at least one of the real-time HRR value of the user at the current time point or at the previous time point, the physical energy level of the user at the previous time point (t-1) or the physiological conditions of the user. In some example, a greater real-time HRR value of the user at the current time point (t) or at the previous time point (t-1) is associated with a greater physical energy recovery rate of the user. For instance, the second physical energy recovery rate of the user has an exponential dependency relationship with the real-time HRR value of the user at the current time point (t). In some other examples, the second physical energy recovery rate of the user may be determined based on the physical energy level of the user at the previous time point. For instance, a greater the physical energy level of the user at the previous time point (t-1) is associated with a greater physical energy recovery rate of the user. In some other examples, the second physical energy recovery rate of the user may be determined based on the real-time HRR of the user at the current time point or at the previous time point, as well as the physical energy level of the user at the previous time point. In some other examples, the second physical energy recovery rate of the user is a preset value.

Based upon the physical energy change determined above in accordance with one of the three scenarios (e.g., based upon one or more of the aforementioned hypotheses), the physical energy level of the user at the current time point (t) may be determined at 422 based upon the physical energy consumption or recovery of the user. Thus, based on the method 400 described above, the physical energy 306 of the user may be determined (e.g., calculated) to assess whether the user is in a state of recovery or rest, and thereby provide one of the components utilized to determine the overall readiness 300 (e.g., readiness score) of the user. In some examples, whether the user is in a state of physical energy consumption or physical energy recovery is determined by comparing the real-time HRR value of the user with the CP and comparing the activity level with the threshold. For example, if the real-time HRR value of the user is greater than or equal to the CP, and the activity level of the user is greater than or equal to the threshold, it is determined that the user is in the state of physical energy consumption. For another example, if the real-time HRR value of the user is less than the CP, and/or the activity level of the user is less than the threshold, it is determined that the user is in the state of physical energy recovery. In some other examples, the state of physical energy of the user may be determined by comparing the HRR value of the user with the CP. For example, if the real-time HRR value of the user is greater than or equal to the CP, it is determined that the user is in the state of physical energy depletion. For another example, if the real-time HRR value of the user is less than the CP, it is determined that the user is in the state of physical energy recovery. In this case, optionally, the rate of physical energy consumption and/or physical energy recovery may be determined by comparing the activity level of the user with a corresponding threshold. In some instances, if the activity level of the user is greater than or equal to the corresponding threshold, a relatively high physical energy consumption rate may be determined (or a relatively low physical energy recovery rate may be determined). In some other instances, if the activity level of the user is less than the corresponding threshold, a relatively low physical energy consumption rate may be determined (or a relatively high physical energy recovery rate may be determined). In some other examples, the state of physical energy of the user may be determined by comparing the activity level of the user with the threshold. For example, if the activity level of the user is greater than or equal to the threshold, it is determined that the user is in the state of physical energy depletion. For another example, if the activity level of the user is less than the threshold, it is determined that the user is in the state of physical energy recovery.

In some other examples, the state of physical energy of the user may be determined in other ways, which will not be detailed herein.

In some examples, the real-time HRR value and/or activity level determined at multiple time points may be considered in determining the state of physical energy of the user. For instance, the real-time HRR value at the current time point is compared to that at one or more previous time points to determine a real-time HRR change, and the real-time HRR change is used for determining at least one of the state of physical energy of the user or the current physical energy level of the user.

In the above examples, multiple physical energy recovery mechanisms or models are set, where different physical energy recovery mechanisms or models correspond to different physical energy recovery rates. In some embodiments, different physical energy recovery mechanisms or models may correspond to different algorithms and/or different parameters, which are not limited herein.

In addition, in the above examples, a corresponding physical energy recovery mechanism or model is selected based on the user's real-time HRR (e.g., a comparison between the real-time HRR and an associated threshold). In some other examples, a corresponding physical energy recovery mechanism or model may further or alternatively be selected based on other physiological conditions of the user, which are not limited herein.

In some implementations, the mental energy 308 of the user may be determined based on a determination of whether the user is sleeping. For example, if the user is sleeping, mental recovery of the user is more likely to occur. For another example, if the user is not sleeping (e.g., if the user is awake), mental depleting of the user is more likely to occur. In some implementations, the mental energy 308 of the user may be determined based on the stress level of the user. For example, if the stress level of the user is relatively low, mental recovery of the user is more likely to occur. For another example, if the stress level of the user is relatively high, mental depleting of the user is more likely to occur. In some implementations, the mental energy 308 of the user may be determined based on other physiological conditions of the user.

To further illustrate the mental energy 308 of the user, FIG. 5 illustrates a schematic flow diagram of an example of a method 500 for dynamically determining the mental energy 308 of the user. The mental energy 308 of the user may be determined based on periods of stress 320 and period of recovery 322 of the user, whereby the user may alternate between the periods of stress 320 (i.e., periods of mental stress) and the periods of recovery 322 (i.e., periods of mental recovery). In some examples, the mental energy 308 may also be based on, or take into account, an intensity of the stress 320 (e.g., a level of pressure) since different stress levels (e.g., different pressure levels) may consume different amounts of the user's mental energy, thereby depleting the overall mental capacity (e.g., the readiness) of the user.

By way of example, the mental energy 308 may be determined (e.g., calculated or estimated) based on the SAFTE model, which may predict the effects of at least one of sleep metrics, circadian rhythms, or cognitive load (e.g., stress level) on the user's mental fatigue and/or recovery. In some examples, mental energy consumption (i.e., mental energy depletion) may occur when the pressure level of the stress 320 (i.e., the intensity of the stress 320) is higher than a predefined threshold. In some other examples, mental energy recovery (i.e., mental energy repletion) may occur when the pressure level of the stress 320 is lower than the predefined threshold. Such determinations may be made based on one or more activity metrics and/or one or more sleep metrics of the user (e.g., activity and/or sleep data obtained by the device 100 when worn by the user or obtained by another portable device of the user).

The method 500 may initially start with a mental energy level of the user at a previous time point (t-1) at 502. The method of determining (e.g., calculating) a mental energy level of the user at a current time point (t) will be discussed in further detail below. Based upon the mental energy level of the user at the previous time point (t-1), it may be determined at 504 whether there is a pressure deficit. That is, it may be determined at 504 whether the pressure level of the stress 320 at the current time point (t) is less than the pressure level of the stress 320 at the previous time point (t-1). It should be noted that the pressure level at a particular point in time (e.g., t or t-1) may be determined in any manner. For example, the pressure level may be based upon one or more physiological conditions of the user (e.g., heart rate, heart rate variability, blood pressure, body temperature, physical movement, etc.), whereby the one or more physiological conditions of the user may be based upon one or more physiological signals detected by the device 100 or another portable device of the user.

If a pressure deficit is determined at 504 based upon a comparison of the pressure value at the current time point (t) to the pressure value at the previous time point (t-1), the real-time pressure value of the user may be set at 506 as equal to a preset threshold value. The predefined threshold value may be any desired value. If a pressure deficit is not determined at 504, the real-time pressure value may remain the same as the value calculated (e.g., not modified to be set at the threshold value at 506).

A circadian rhythm of the user may be calculated at 508.The circadian rhythm of the user may be calculated based on the current time point (t) and/or the current moment (e.g., the time of day), a peak or peak point offset of 24-hour rhythm, or a combination thereof.

A sleep propensity of the user may be calculated at 510. The sleep propensity (e.g., a sleep tendency) of the user may be calculated based on the circadian rhythm determined at 508 as described above. In some other examples, the sleep propensity of the user may be calculated based on one or more of the user's personal data, history sleep data, or the like.

A sleep debt of the user may be calculated at 512. The sleep debt of the user may be the difference between the amount of sleep the user needs and the amount of sleep the user gets. In some examples, the sleep debt of the user may be determined based on a cognitive energy reserve (or mental energy reserve) of the user at the current time point (t). In some examples, the sleep debt of the user may be negatively correlated with the cognitive energy reserve of the user at the current time point. For instance, a greater cognitive energy reserve of the user at the current time point may be associated with a smaller sleep debt of the user. For another instance, a smaller cognitive energy reserve of the user at the current time point may be associated with a greater sleep debt of the user.

A sleep intensity of the user may be calculated at 514. The sleep intensity may be an indication of the intensity (e.g., quality) of the sleep of the user and may be determined based on at least one of the sleep propensity determined at 510 as described above, the sleep debt determined at 512 as described above, or a real-time stress or pressure value at the current time point (t). For example, the sleep intensity of the user is determined based on a difference between the real-time pressure value of the user at the current time point (t) and a pressure threshold (e.g., the threshold value utilized at 506). In some examples, the sleep intensity of the user may be negatively correlated with the real-time stress value of the user. For instance, a lower real-time stress value of the user may correspond to a higher sleep intensity of the user, and vice versa. For another instance, the sleep intensity of the user is determined based on an accumulation of the sleep propensity and the sleep debt. For another example, the sleep intensity of the user may be positively correlated with a sum of the sleep propensity and the sleep debt of the user. For another example, the sleep intensity of the user at the current time point may be determined based on the sleep propensity, sleep debt, and real-time stress value of the user at the current time point.

It may be determined whether the user is sleeping (i.e., whether the user is in a state of sleeping) at 516. Determining whether the user is sleeping may be based upon motion data, heart rate data, one or more of the factors calculated at 508-514 (e.g., the circadian rhythm, the sleep propensity, the sleep debt, and the sleep intensity), the pressure level of the stress 320 experienced by the user, other factors (e.g., one or more physiological conditions of the user, which may be based upon one or more physiological signals detected by the device 100 or another portable device of the user), or any combination thereof.

By way of example, determining whether the user is sleeping at 516 may be based upon an evaluation of the cognitive reserve of the user at the current time point (t), whereby the evaluation of the cognitive reserve of the user at the current time point (t) may be based upon the cognitive reserve of the user at the previous time point (t-1), the sleep intensity of the user calculated at 514 as described above, or both. For example, the cognitive reserve of the user at the current time point may be a sum of the cognitive reserve of the user at the previous time point and the sleep intensity. For another example, the cognitive reserve of the user at the current time point may be a weighted sum of the cognitive reserve of the user at the previous time point and the sleep intensity, where the weights may be fixed values, or the weights may be dynamically determined based on one or more sleep parameters of the user (e.g., sleep intensity, real-time sleep quality, real-time sleep time duration, real-time sleep stage or any combination thereof).

If it is determined at 516 that the user is sleeping (i.e., the user is in the state of sleeping), it may be determined that the user is in the state of mental energy recovery at 518. In this case, the mental energy level of the user may be determined or updated based on a nighttime mental energy recovery mechanism or model, where the nighttime mental energy recovery mechanism or model may be associated with a relatively high mental energy recovery rate. The mental energy level of the user at the current point time (t) at 518 may be determined based on the cognitive reserve of the user at the current time point (t). That is, the mental energy level of the user may be determined at 518 for the current time point (t) based upon a cognitive reserve of the user that reflects that the user is sleeping. For example, the mental energy level of the user is the same as the cognitive reserve of the user. For another example, the cognitive reserve of the user is mapped to the mental energy level of the user by a predefined algorithm. In some examples, the mental energy level of the user at the current time point may be positively correlated with the cognitive reserve of the user at the current time point. For instance, the mental energy level of the user at the current time point may have a linear dependency relationship with the cognitive reserve of the user at the current time point.

If it is determined at 516 that the user is not sleeping (i.e., the user is not in the state of sleeping), the pressure level of the stress 320 experienced by the user may be evaluated at 520 to determine whether the pressure (e.g., the current pressure level) is greater than or equal to the preset threshold (e.g., the preset pressure threshold value).

If it is determined at 520 that the current pressure level is less than the preset pressure threshold value, it may be determined that the user is in the state of mental energy recovery, and the mental energy level of the user may be determined or updated in accordance with a daytime mental energy recovery mechanism or model, as indicated along the lead line from 520 to 518 in FIG. 5, where the daytime mental energy recovery mechanism or model may be associated with a relatively low mental energy recovery rate. In particular, the mental energy level of the user at the current time point (t) may be determined based on the cognitive reserve of the user at the current time point (t), whereby the cognitive reserve of the user at the current time point (t) may be determined based on at least one of the cognitive reserve of the user at the previous time point (t-1), the sleep intensity of the user calculated at 514 as described above, the pressure threshold, or the real-time pressure value. Thus, the mental energy level of the user may take into account that the pressure level experienced by the user is less intense (e.g., less than the predefined threshold), and the mental energy repletion is relatively slow. For example, the mental energy level (or the cognitive reserve) of the user is determined based on a difference between the current pressure level (i.e., the real-time pressure value at the current time point) of the user and the pressure threshold. Optionally, the mental energy level (or the cognitive reserve) of the user may be positively correlated with a difference between the current pressure level of the user and the pressure threshold. For another example, the mental energy level (or the cognitive reserve) of the user is determined based on the mental energy reserve (i.e., the mental energy level or cognitive reserve of the user at the previous time point) of the user and a mental energy recovery rate (or a cognitive reserve recovery rate) associated with the daytime mental energy recovery mechanism or model. The mental energy recovery rate (or the cognitive reserve recovery rate) may be a preset value, or may be determined based on the current pressure level or other physiological conditions of the user. In some examples, the mental energy recovery rate (or the cognitive reserve recovery rate) may be based on at least one of the sleep intensity or the current pressure level of the user. In some other examples, the mental energy recovery rate (or the cognitive reserve recovery rate) may be based on a difference between the current pressure level of the user and a pressure threshold. For instance, a greater difference between the current pressure level of the user and the pressure threshold is associated with a higher mental energy recovery rate (or the cognitive reserve recovery rate). For another instance, a greater sleep intensity results in a higher mental energy recovery rate (or the cognitive reserve recovery rate).

If it is determined at 520 that the current pressure level is greater than or equal to the preset pressure threshold value, it may be determined that the user is in the state of mental energy depletion/consumption. In this case, the mental energy level of the user may be determined in accordance with a daytime mental energy depletion mechanism or model, as indicated by the additional lead line from 520 to 518 in FIG. 5. In particular, the mental energy level at the current time point (t) may be determined using the cognitive reserve at the current time point (t) as described above, whereby the cognitive reserve at the current time point (t) may be determined based on one or more of the cognitive reserve of the user at the previous time point (t-1) and the real-time pressure value. Thus, the mental energy level of the user may take into account that the pressure level experienced by the user is more intense (e.g., greater than or equal to the predefined threshold). In some examples, the cognitive reserve of the user at the current time point may be determined based on the cognitive reserve of the user at the previous time point and the real-time pressure value at the current time point or at the previous time point, such as a difference between the cognitive reserve at the previous time point and a cognitive reserve consumption based on the real-time pressure value at the current time point. In some examples, the cognitive reserve of the user at the current time point may be determined based on the cognitive reserve of the user at the previous time point and a cognitive reserve consumption rate in accordance with the daytime mental energy depletion mechanism or model, where the cognitive reserve consumption rate may be determined based on the user's personal data, a default value or the history physiological data of the user such as one or any combination of history pressure data, history sleep data, or the like. In some examples, the cognitive reserve or cognitive reserve consumption rate at the current time point may be determined based on a difference between the real-time pressure value at the current time point and the pressure threshold value. In some examples, the cognitive reserve consumption rate may be positively correlated with the difference between the real-time pressure value and the pressure threshold value, such as based on a linear function, which is not limited herein.

In some examples, based on the method 500 described above, the mental energy 308 of the user may be determined (e.g., calculated or estimated) based upon the mental energy level (or the cognitive reserve) of the user at the current time point and the mental energy level (or the cognitive reserve) of the user at the previous time point (e.g., a comparison thereof to determine whether mental recovery of the user exists). The mental energy 308 of the user determined may provide the components utilized to determine the overall readiness 300 (e.g., readiness score) of the user.

In the above examples, multiple mental energy recovery mechanisms or models are set, where different mental energy recovery mechanisms or models correspond to different mental energy recovery rates. In some embodiments, different mental energy recovery mechanisms or models may correspond to different algorithms and/or different parameters, which are not limited herein.

In addition, in the above examples, a corresponding mental energy recovery mechanism or model is selected based on whether the user is sleeping. In some other examples, a corresponding mental energy recovery mechanism or model may further or alternatively be selected based on other physiological conditions of the user. For instance, it may be selected based on other sleep conditions of the user (such as sleep quality, current sleep stage the user, sleep interruptions, continuous sleep time duration, etc.), which is not limited herein.

Based on the components shown in FIG. 3 (e.g., the physical energy 306 determined based on the method 400 shown in FIG. 4, the mental energy 308 determined based on the method 500 shown in FIG. 5, the breathing health 310 of the user, the heart health 312 of the user, and the body temperature 314 of the user), the readiness 300 of the user (e.g., the readiness score associated with the user) may be determined.

By way of example, the readiness score associated with the readiness 300 of the user may be determined by first determining a readiness component score for each of the components described above (e.g., one or more of the components shown in FIGS. 3-5). For example, based upon the factors of each of the components and/or the calculations described above (e.g., the method 400 to determine the physical energy 306 (e.g., to determine physical recovery of the user) and the method 500 to determine the mental energy 308 (e.g., to determine mental recovery of the user)), each of the components may be assessed to determine a readiness component score. The readiness component score of each component may be or may be converted into a numerical value from 0 to 100. If conversion of the readiness component scores is required to convert the value into a numerical value from 0 to 100, any conversion method may be utilized. For example, the physical energy level as mentioned above may be used as the readiness component score corresponding to the physical energy 306, and/or the mental energy level as mentioned above may be used as the readiness component score corresponding to the mental energy 308. For another example, the physical energy level and/or mental energy level as mentioned above may be processed by one or more processing (such as mapping or conversion) to obtain corresponding readiness component scores.

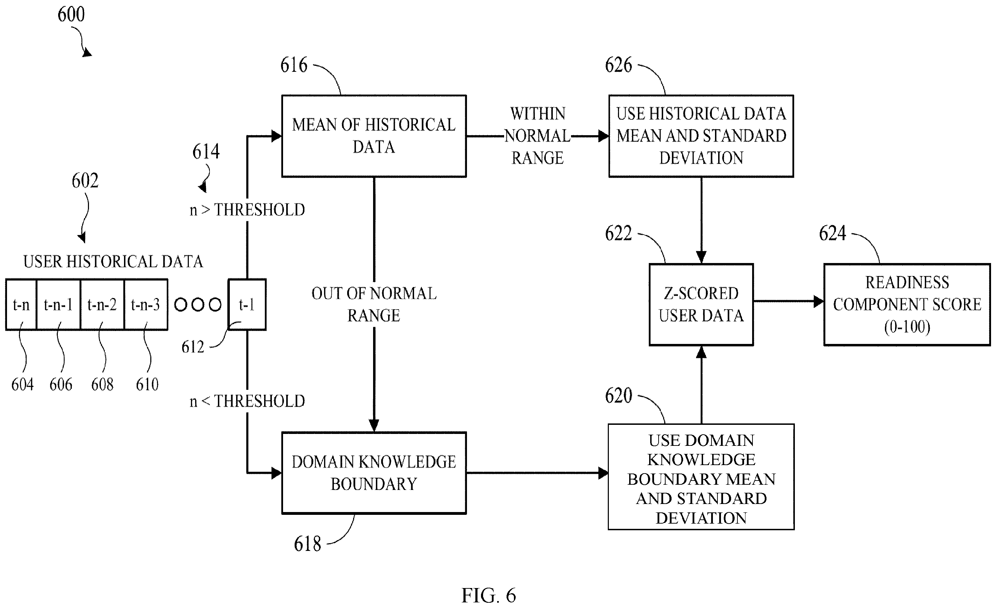

FIG. 6 illustrates a schematic flow diagram of an example of a method 600 for dynamically determine a readiness component score for one or more of the components described above. For example, the method 600 may be used to obtain an assessment of at least one component of the breathing health, heart health, or body temperature. As shown in FIG. 6, each of the components described above may be assessed according to user historical data to calculate individualized statistical measures of the readiness component scores for the component. Moreover, the method 600 may also account insufficient historical data and/or deviation from a normal value range, as described in further detail below.

To further illustrate how any component utilized to determine the readiness 300 of the user may be assessed to determine a readiness component score, FIG. 6 will now be described in further detail. It should be noted that the generic term "component" will be used with respect to FIG. 6, which may represent any one or more of the components described above with respect to FIGS. 3-5, unless otherwise stated.

As described above, user historical data 602 may be used to assess the component to determine the readiness component score of the component. For example, as shown in FIG. 6, the user historical data 602 may include one or more historical data points (e.g., data values) for different time points (e.g., represented as t-n, t-n-1, t-n-2, t-n-3, where t represents the current time point (t) and n represents the total number of historical data points (e.g., a total number of days, whereby each historical data point is taken on a different day)). The historical data points are illustrated as 604-612 in FIG. 6. It should be noted that the historical data points may represent one or more desired timescales. For example, the historical data points may be taken to represent a daily, weekly, monthly, or annual timescale of data points.

As discussed above, the assessment of the component may be done according to the user historical data 602 to calculate the readiness component score of the component if there is sufficient user historical data and/or the historical data points are determined to fall within a normal range (e.g., a predefined normal range). To determine whether there is sufficient user historical data, the user historical data 602 may evaluated to determine whether the total number n of historical data points (e.g., the total number of days, whereby each day may include a data point) is greater than or less than a predefined threshold at 614. The predefined threshold may be any desired value. For example, in the case where a data point of the condition is determined on a daily basis (e.g., n is equal to the number of days of evaluation of the user), the predefined threshold may be a month (e.g., the predefined threshold is equal to 30 days).

If it is determined that the total number n of historical data points is greater than the predefined threshold at 614, the method 600 may then calculate a mean of the user historical data 602 at 616. However, if it is determined that the total number n of historical data points is less than the predefined threshold at 614, the method 600 may then determine domain knowledge boundaries at 618. The domain knowledge boundaries at 618 may be predefined boundaries utilized to determine the readiness component score of the component. That is, the domain knowledge boundaries may be limits or edges of possible values of the user data that may be used in lieu of the user historical data 602 (e.g., where there is insufficient user historical data). In some examples, similarly, if the mean of user historical data determined at 616 is out of the normal range (e.g., the normal predefined range), domain knowledge boundaries may be determined at 618. Thus, the method 600 may take into account both insufficient user historical data and significant deviation from the normal range in the user historical data.

Once the domain knowledge boundary is determined at 618, a statistic metric, such as mean and standard deviation, of the domain knowledge boundary may be determined at 620. Based upon the statistic metric, such as the mean and standard deviation, of the domain knowledge boundary determined at 620, a Z-score of the user data may be determined (e.g., calculated) at 622. The Z-score of the user data may correspond to a statistical measurement that described how many standard deviations the user data (e.g., a plurality of data points of the user data) is from the mean, thereby facilitating a standardized statistic metric for any of the components. That is, based on the Z-score determined at 622, a readiness component score in the range of 0 to 100 may be determined at 624.

As described above, in some examples, the readiness component score determined at 624 may be based on the domain knowledge boundary determined at 618 and the statistic metric, such as the mean and standard deviation of the domain knowledge boundary determined at 620 when the user historical data 602 is insufficient or deviates too significantly. However, in some other examples, when the user historical data 602 is determined to be sufficient (e.g., the total number n of historical data points is greater than the predefined threshold) at 614 and the mean of the historical data determined at 616 is within the normal range, the readiness component score determined at 624 may be based on the user historical data 602.

For example, when the mean of the user historical data 602 determined at 616 is within the normal range, the statistic metric of the user historical data 602, such as the mean of the user historical data 602 and/or a standard deviation of the user historical data 602 determined at 626 may be used to determine the readiness component score. That is, the Z-score determined

(e.g., calculated) at 622 may be based upon the statistic metric of the user historical data 602 instead of the statistic metric of the domain knowledge boundary. The Z-score determined at 622 based upon the user historical data 602 may then be used to determine the readiness component score of the component at 624.

Thus, using the method 600 to determine the readiness component score of each of the components, the overall readiness score associated with the readiness 300 of the user may be based upon standardized readiness component scores of the components. In particular, the readiness score associated with the readiness 300 of the user (also referred to as the final readiness score) may be a weighted average of the readiness component scores determined for one or more of the components in accordance with the method 600 described above. That is, the readiness score may be determined based on the readiness component scores associated with any one of the components described above. In some examples, one or more weight values for the components may be determined in accordance with their associated readiness component scores, e.g., a smaller weight value is determined for a greater readiness component scores associated with the components. In some examples, one or more weight values for the components are determined based on one or any combination of the user personal data, user historical data, or the like.

In some embodiments, the readiness score and/or the readiness component scores may be provided to the user in any desired manner (e.g., textually, graphically, numerically, audio, vibration, LED lighting, etc.). For example, the readiness score and/or the readiness component scores may be provided to the user visually via the display of the device 100 or a display of another portable device of the user (e.g., a laptop, tablet, or smartphone). As a result, the user may review the readiness score and/or the readiness component scores to assess their current schedule and/or activities and avoid unwanted burnout that may be caused by physical and/or mental overexertion.

In some embodiments, the readiness score and/or the readiness component scores may be adopted to any desired schedule and/or format. For example, the readiness score may be provided daily to the user based on user readings (e.g., user data associated with the one or more components) from the previous day, whereby the user readings may be used to determine (e.g., calculate or estimate) the various readiness component scores and produce a weighted average or weighted sum that represents the readiness score as described above.

Additionally, in some embodiments, after calculating a daily readiness score, one or more physiological conditions of the user may be monitored (e.g., via monitoring the one or more physiological signals detected by the device 100 when worn by the user) to continuous update one or more of the components based upon physical and/or mental energy expenditure and/or recovery throughout the day. As such, the readiness score of the user may by dynamically monitored and continuously updated in real-time to reflect the present readiness of the user.

Furthermore, in some embodiments, the components determined (e.g., calculated or estimated) based on the one or more physiological signals detected by the device 100 when worn by the user may also be combined with subjective feedback provided by the user. For example, the user may provide feedback (e.g., answer a goal-directed questionnaire or provide their feedback in any desired format) via the device 100 or another portable device of the user to assess the subjective user status. That is, the user may provide feedback to determine the subjective readiness score of the user. Subsequent determinations of a daily or real-time readiness score as described above may integrate the subjective data from the user with the objective data from the device 100 to provide a more tailored, accurate, and personalized readiness score to the user. In some examples, a final readiness score of the user may be obtained based on a readiness score based on the objective data obtained from the at least one sensor as mentioned above (referred to as an objective readiness score) and a readiness score obtained from the subjective feedback of the user (referred to as a subjective readiness score). For instance, the final readiness score of the user may be obtained based on a weighted average of the objective readiness score and the subjective readiness score of the user. For another instance, the objective readiness score may be calibrated or adjusted based on the subjective readiness score of the user, thereby obtaining the final readiness score of the user. In some other examples, by comparing the subjective readiness score with the objective readiness score of the user, one or more parameters for determining the objective readiness score may be adjusted, e.g., the weight of one or more components may be adjusted, or one or more parameters for determining the readiness component scores of one or more components may be adjusted, etc.

In some embodiments, similarly, the user may provide feedback (e.g., answer the goal-directed questionnaire or provide their feedback in any desired format) to assess the subjective user status for one or more of the readiness components (e.g., any of the components described above). That is, the user may provide feedback to determine one or more of the readiness components and/or one or more of the readiness component scores associated with the readiness components. For example, the user may answer subjective questions (e.g., via the device 100) that correspond to one or more of the components shown in FIG. 3 that correspond to the readiness 300 of the user. Thus, current and/or subsequent determinations of a daily or real-time readiness component or readiness component score may integrate the subjective data from the user with the objective data from the device 100. In some examples, a final readiness component score of the user may be obtained based on the readiness component score based on the objective data as mentioned above (referred to as an objective readiness component score) and the readiness component score of the user obtained based on the subjective feedback of the user (referred to as a subjective readiness component score). For instance, the final readiness component score for a component of the user may be obtained based on a weighted average of the objective readiness component score and the subjective readiness component score of the component. For another instance, the objective readiness component score of a component may be calibrated or adjusted based on at least one of the subjective feedback of the user or the subjective readiness component score, thereby obtaining the final readiness component score of the component. In some other examples, by comparing the subjective readiness component score with the objective readiness component score of the user, one or more parameters used to determine the objective readiness component score may be adjusted. In some other examples, a weight associated with the objective readiness component score of a component may be adjusted based on the subjective readiness component score of the component or the subjective feedback of the component from the user. In some other examples, the objective readiness component score of a component may be selected or filtered based on the subjective readiness component score of the component or the subjective feedback of the component from the user, etc., which will not be detailed herein.

Moreover, in some embodiments, the user may be provided guidance based on the readiness score determined. In some examples, the readiness score may be provided to the user (e.g., via the device 100) with expert-curated guidance to accompany the different variations of the readiness score as well as the different combinations of components described above. Such guidance may be or may include information associated with the readiness score and/or the readiness component scores, instructions on how to address an underlying situation associated with the readiness score and/or the readiness component scores, recommendations (e.g., recommended activities or tasks), or a combination thereof. Such guidance may be provided in real-time to the user or based upon a desired duration of time (e.g., daily, weekly, monthly, or yearly).

Additionally, in some embodiments, such guidance may be in the form of an interactive conversation (e.g., a chatbot) with the user. For example, the chatbot may utilize large language models (LLMs) to cure domain knowledge bases and metrics that cover all the components and the various readiness scores. The user may then use natural language (e.g., via voice or text) to ask questions about the domain knowledge or readings related to the components and/or the various readiness scores.

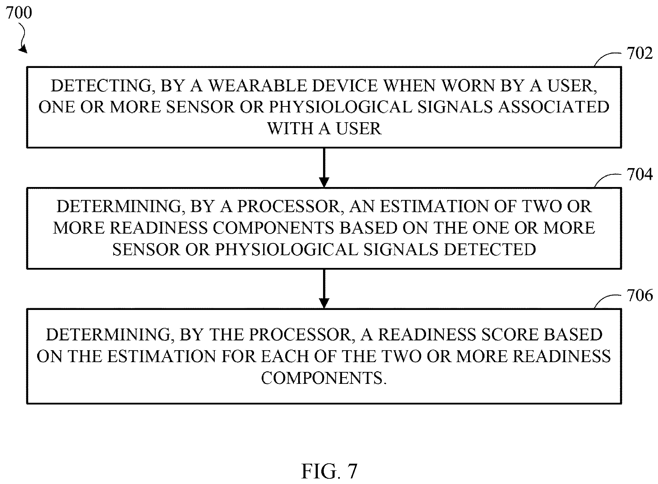

FIG. 7 is a schematic flow diagram of an example of a method 700 for dynamically determining a readiness score of a user using a wearable device. For example, the method 700 shown in FIG. 7 may be implemented using the device 100 and/or one or more external devices in communication with the device 100. The method 700 may be similar to, be part of, or include the method 400, the method 500, the method 600 or a combination thereof.

The method 700 can be implemented as software and/or hardware modules in the computing device 200 of FIG. 2. For example, the method 700 can be implemented as software modules stored in the storage 230 as instructions and/or data executable by the processor of an apparatus, such as the device 100 in FIG. 1. In another example, the method 700 can be implemented in hard as a specialized chip storing instructions executable by the specialized chip. Some or all of the operations of the method 700 can be implemented by the processor 205 of FIG. 2.

At 702, it is detected, by a wearable device (e.g., the device 100) when worn by the user, one or more sensor or physiological signals associated with the user. The one or more sensor or physiological signals may be associated with one or more physiological conditions of the user described above. Additionally, the one or more sensor or physiological signals may be associated with one or more of the components shown in FIG. 3 and described above.

At 704, it is determined, by a processor, an estimation of two or more readiness components based on the one or more sensor or physiological signals detected. The two or more readiness components may be two or more of the components shown in FIG. 3 that may be associated with the readiness 300 of the user. That is, the readiness components may be any of the components utilized to calculate a readiness score of the user. For example, the two or more readiness components may include a physical energy of the user associated with physical energy consumption of the user, physical energy recovery of the user, or both, and a mental energy of the user associated with mental energy consumption of the user, mental energy recovery of the user, or both.

At 706, it is determined, by the processor, a readiness score based on the estimation of each of the two or more readiness components. For example, the readiness score may be determined as described above with respect to FIG. 6 (e.g., determined as a weighted average or weighted sum of the readiness component scores).

Technical specialists skilled in the art should understand that the implementations in this disclosure may be implemented as methods, systems, or computer program products. Therefore, this disclosure may be implemented in forms of a complete hardware implementation, a complete software implementation, and a combination of software and hardware implementation. Further, this disclosure may be embodied as a form of one or more computer program products which are embodied as computer executable program codes in computer writable storage media (including but not limited to disk storage and optical storage).

This disclosure is described in accordance with the methods, devices (systems), and flowcharts and/or block diagrams of computer program products of the implementations, which should be comprehended as each flow and/or block of the flowcharts and/or block diagrams implemented by computer program instructions, and the combinations of flows and/or blocks in the flowcharts and/or block diagrams. The computer program instructions therein may be provided to generic computers, special-purpose computers, embedded computers or other processors of programmable data processing devices to produce a machine, wherein the instructions executed by the computers or the other processors of programmable data processing devices produce an apparatus for implementing the functions designated by one or more flows in the flowcharts and/or one or more blocks in the block diagrams.

The computer program instructions may be also stored in a computer readable storage which is able to boot a computer or other programmable data processing device to a specific work mode, wherein the instructions stored in the computer readable storage produce a manufactured product containing the instruction devices which implements the functions designated by one or more flows in the flowcharts and/or one or more blocks in the block diagrams.

The computer program instructions may also be loaded to a computer or another programmable data processing device to execute a series of operating procedures in the computer or the other programmable data processing device to produce a process implemented by the computer, whereby the computer program instructions executed in the computer or the other programmable data processing device provide the operating procedures for the functions designated by one or more flows in the flowcharts and/or one or more blocks in the block diagrams.

Apparently, the technical specialists skilled in the art may perform any variation and/or modification to this disclosure by the principles and within the scope of this disclosure. Therefore, if the variations and modifications herein are within the scope of the claims and other equivalent techniques herein, this disclosure intends to include the variations and modifications thereof.

Unless defined otherwise, all technical and scientific terms used herein have the same meaning as commonly understood by one of ordinary skill in the art. Methods and materials similar or equivalent to those described herein can be used in the practice or testing of the present disclosure. As used in the specification, and in the appended claims, the singular forms "a," "an," "the" include plural referents unless the context clearly dictates otherwise. The term "comprising," and variations thereof as used herein is used synonymously with the term "including" and variations thereof and are open, non-limiting terms. The terms "optional" or "optionally" used herein mean that the subsequently described feature, event or circumstance may or may not occur, and that the description includes instances where said feature, event or circumstance occurs and instances where it does not. The terms "at least one of A or B," "at least one of A and B," "one or more of A or B," "A and/or B" used herein mean "A," or "B" or "A and B."

While the disclosure has been described in connection with certain embodiments or implementations, it is to be understood that the disclosure is not to be limited to the disclosed embodiments but, on the contrary, is intended to cover various modifications and equivalent arrangements included within the scope of the appended claims, which scope is to be accorded the broadest interpretation so as to encompass all such modifications and equivalent structures as is permitted under the law.

## Claims

1. A method of dynamically determining a readiness score of a user using a wearable device, comprising:
acquiring, by a processor, one or more sensor or physiological signals associated with the user detected by the wearable device when worn by the user;
determining, by the processor, an estimation of two or more readiness components based on the one or more sensor or physiological signals, wherein the two or more readiness components comprise:
a physical energy of the user associated with physical energy consumption of the user, physical energy recovery of the user, or both; and
a mental energy of the user associated with mental energy consumption of the user, mental energy recovery of the user, or both; and
determining, by the processor, the readiness score based on the estimation of each of the two or more readiness components.

2. The method of claim 1, wherein the physical energy of the user is determined based on a physical energy reserve of the user, one or more heart-related metrics of the user, and one or more motion-related metrics, and wherein the one or more heart-related metrics comprise a real-time heart rate reserve.

3. The method of claim 1 or 2, wherein determining the estimation of the two or more readiness components based on the one or more sensor or physiological signals, comprises:
determining at least one of a real-time heart rate or an activity level of the user based on the one or more sensor or physiological signals;
determining that the user is in a state of physical energy consumption or a state of physical energy recovery based on at least one of the real-time heart rate or the activity level of the user.

4. The method of any one of claims 1 to 3, wherein determining the estimation of the two or more readiness components based on the one or more sensor or physiological signals, comprises:
based on the one or more sensor or physiological signals, determining a physical energy recovery rate of the user at a current time point, and/or selecting a corresponding physical energy recovery model from a plurality of preset physical energy recovery models, wherein the plurality of preset physical energy recovery models correspond to different physical energy recovery rates.

5. The method of any one of claims 1 to 4, wherein the mental energy of the user is determined based at least on a real-time stress of the user obtained from the one or more sensor or physiological signals and a mental energy reserve of the user.

6. The method of any one of claims 1 to 5, wherein determining the estimation of the two or more readiness components based on the one or more sensor or physiological signals comprises:
determining whether the user is in a state of sleeping or not based on the one or more sensor or physiological signals; and
based on whether the user is in the state of sleeping or not, determining that the user is in a state of mental energy consumption or a state of mental energy recovery, and/or, determining or updating the mental energy of the user.

7. The method of any one of claims 1 to 6, wherein determining the estimation of the two or more readiness components based on the one or more sensor or physiological signals comprises:
determining whether the user is in a state of mental energy consumption or a state of mental energy recovery based at least in part on a stress level associated with a severity of stress obtained from the one or more sensor or physiological signals.

8. The method of any one of claims 1 to 7, wherein determining the estimation of the two or more readiness components based on the one or more sensor or physiological signals comprises:
based on the one or more sensor or physiological signals, determining a mental energy recovery rate of the user at a current time point, and/or determining a corresponding mental energy recovery model from a plurality of preset mental energy recovery models, wherein the plurality of preset mental energy recovery models correspond to different mental energy recovery rates.

9. The method of any one of claims 1 to 8, wherein the two or more readiness components further comprise at least one of heart health, breathing health, and body temperature.

10. The method of any one of claims 1 to 9, wherein the estimation of each of the two or more readiness components is determined based on historical sensor or physiological data such that the estimation of each of the two or more readiness components is individualized for the user, and wherein the historical sensor or physiological data is tracked based one or more timescales, the one or more timescales comprising at least one of a daily timescale, a weekly timescale, and a monthly timescale.

11. The method of any one of claims 1 to 10, wherein the readiness score is determined based on the estimations of the two or more readiness components as well as weights of the two or more readiness components, and wherein the weights of the two of more readiness components are determined based on the estimations for the two or more readiness components.

12. The method of any one of claims 1 to 11, further comprising:
acquiring, by the processor, a subjective estimation of at least one of the readiness score, or one or more of the readiness components, input by the user; and
adjusting, by the processor, the readiness score based on the subjective estimation of the readiness score, the estimation of the one or more of the readiness components based on the subjective estimation of the one or more of the readiness components, or both.

13. The method of any one of claims 1 to 12, further comprising:
providing guidance to the user based on the readiness score, wherein the guidance comprises at least one of information associated with the readiness score, information associated with the estimation of each of the two or more readiness components, instructions on how to address an underlying situation associated with the readiness score, or recommended activities.

14. An electronic device for dynamically determining a readiness score of a user using a wearable device, comprising:
a non-transitory memory; and
a processor configured to execute instructions stored in the non-transitory memory to implement the method of any one of claims 1 to 13.

15. A non-transitory computer-readable storage medium configured to store computer programs for dynamically determining a readiness score of a user using a wearable device, the computer programs comprising instructions executable by a processor to implement the method of any one of claims 1 to 13.
